# EUROPEAN PATENT APPLICATION

(11) **EP 1 811 029 A1**
(43) Date of publication of application: **25.07.2007**
(21) Application number: 05799974.0
(22) Date of filing: 02.11.2005
(51) Int. Cl.: C12N 15/09, C12N 9/02

(54) **METHOD OF ISOLATING P450 GENE**

(30) Priority: 10.11.2004 JP 2004326139; 23.03.2005 JP 2005083019; 23.03.2005 JP 2005083122
(71) Applicant: MARINE BIOTECHNOLOGY INSTITUTE CO., LTD., Kamaishi-shi, Iwate 026-0001 (JP)
(72) Inventor: KUBOTA, Mitsutoshi,, Tokyo 1920373 (JP); NODATE, Miho, MARINE BIOTECH. INSTITUTE CO., LTD., Kamaishi-shi Iwate 026-0001 (JP); UCHIYAMA, Taku,, Sakaemachi, Inba-gun, Chiba 2701516 (JP); MISAWA, Norihiko, MARINE BIOTECH.INST. CO., LTD., Kamaishi-shi, Iwate 026-0001 (JP)
(74) Representative: Woods, Geoffrey Corlett
(86) International application number: PCT/JP2005/020180
(87) International publication number: WO 2006/051729

(57) **Abstract**

The present invention provides a method for preparing a hybrid gene. The method includes a step of amplifying a P450 gene fragment contained in a sample using primers designed on the basis of regions of a plurality of P450 in which amino acid sequences are highly conserved and a step of preparing the hybrid gene using the amplified fragments and a known P450 gene. The method includes no culturing step or a step of normalizing extracted DNAs and is useful in isolating a P450 gene from various microbial resources.

The present invention further provides a fused cytochrome P450 monooxygenase containing a peptide which is linked to the C-terminus of a P450 protein with a linker portion disposed therebetween and which has the same function as that of a reductase domain contained in a cytochrome P450 monooxygenase originating from Rhodococcus sp. strain NCIMB 9784. This enables the construction of a high-efficiency electron transfer system useful for various P450 proteins and also enables the production of an active P450 monooxygenase.

## Description

### Technical Field

The present invention relates to a method for isolating a gene encoding a protein serving as a cytochrome P450 (P450), oligonucleotides useful for the method, a novel gene isolated by the method, a protein encoded by the gene, a fused P450 monooxygenase containing the protein, and a method for producing an oxidized compound using the enzyme. The isolation method according to the present invention can be used to isolate an enzyme gene without isolating microorganisms such as bacteria from seawater samples or soil samples, without cultivating such microorganisms, or without normalizing DNA samples extracted from the cultivated or isolated microorganisms. In particular, the isolation method is useful in isolating a novel P450 gene from genetic resources in the natural environment in the case that only a small number of microorganisms having a target gene are present in the genetic resources. P450 synthesized from a gene obtained by the isolation method according to the present invention can be used as a biocatalyst for producing products useful for the chemical industry.

### Background Art

A cytochrome P450 monooxygenase is present in various organisms such as bacteria, plants, and mammals and is an iron- and sulfur-containing enzyme that catalyzes a reaction (monooxygenation) that produces an oxidation product in such a manner that one atom of an oxygen molecule is reduced into one mole of water and the other one is introduced in to a low-molecular-weight organic compound. Although the molecular structure and catalytic function of P450s are essentially not varied, the structure of a substrate-binding site thereof is varied. Hence, P450s are suitable for not only the hydroxylation of a hydrocarbon but also various oxidation reactions such as dealkylation, epoxidation, C-C bond cleavage, aromatization, and dehydrogenation. Examples of the reaction catalyzed by P450s include various biochemical reactions such as the biosynthesis of animal hormones and secondary plant metabolites, the activation of foreign substances in organisms, and the biodegradation of such foreign substances. Since P450s are involved in the oxidation of various organic compounds, bio-industries have investigated P450s. In particular, the following techniques have been being investigated: the synthesis of new low-molecular-weight organic compounds and the decomposition of environmentally hazardous substances by the catalysis of P450, the compounds and substances being hardly oxidized by known methods. In view of industry, for the purpose of the application of bioconversion, the use of P450s originating from microorganisms such as bacteria has been recently disclosed (Non-patent Document 1). Examples of bacterial P450s usually investigated include P450cam originating from Pseudomonas putida and P450BM3 originating from Bacillus megaterium. P450cam is the first of which the crystal structure has been elucidated. P450BM3 has a structure fused with a reductase necessary for the oxidation of the P450. For these enzymes, attempts have been made to improve their activity and/or substrate specificity by molecular evolution engineering, i. e., site-directed mutagenesis (Non-patent Document 1). However, the number of types of P450s originating from known bacteria is extremely less as compared to the diversity of the bacteria. On the other hand, recent advances in genome analysis have offered new possibilities for P450 investigation. That is, the completion of the genome analysis of various bacteria has revealed the presence of various types of bacterial P450s. For example, Mycobacterium tuberculosis has 20 types of P450 sequences and Streptomyces coelicolor has 18 types of P450 sequences. Attempts have been made to use these novel P450 sequences as oxidase resources for bioconversion. Although various types of P450s have been isolated and analyzed in recent years, P450s can be obtained only from microorganisms that can be isolated and cultivated. However, the number of species of such isolatable, cultivable microorganisms is 1% or less of that of microorganisms present in soil or sea (Non-patent Document 2). Hence, the number of types of identified microbial P450s is limited and various types of P450s that are difficult to isolate or identify are probably present in environments. It cannot be denied that unidentified P450s includes ones involved in the oxidation of various organic compounds. In view of industry, it is very worthy to directly isolate various genes, such as P450 genes originating from microorganisms (uncultivable microorganisms) that cannot be isolated or cultivated or are difficult to isolate or cultivate, from the environments to analyze their functions quickly.

A method called cassette PCR is an example of a technique for isolating or recovering a target gene from the environment without isolating or cultivating microorganisms and has been developed by Okuta et al. (Non-patent Document 3). This method is as follows: from a known protein with a target function or an amino acid sequence or nucleotide sequence of a gene encoding the protein, a primer specific to the gene is designed; DNA amplification such as PCR is performed using a DNA, directly extracted from an environmental sample such as a soil sample or a brine sample, as a template; obtained DNA fragments are isolated; and both ends of the gene that cannot be amplified using the primer are supplemented with nucleotide sequences of a known gene similar to that gene. This allows the formation of a hybrid gene including a new gene sequence originating from the DNA directly extracted from the environment sample and known gene sequences located at both ends of the hybrid gene, the new gene sequence being located at a center region of the hybrid gene. Therefore, if the hybrid gene is inserted into a protein expression system parasitic on Escherichia coli, functions of an obtained gene can be efficiently analyzed. The homology between a sequence of a primer used for DNA amplification and an existing protein or gene sequence used to obtain basic information for designing the primer is critical in obtaining various novel gene sequences by this method. However, since P450s have various primary structures, any primer useful in efficiently isolating diverse P450 genes from known sequences has not been obtained yet. In the direct recovery of DNA from an environmental sample such as a soil sample, a large number of DNA fragments originating from major microorganisms of which the number is huge are obtained. If a library is prepared using the DNA fragments, the library contains a large number of genes originating from the major microorganisms. Therefore, when a target gene is in microorganisms (minor microorganisms) of which the number is small, the isolation of this gene is difficult. A technique for sequring the diversity of DNAs of genes originating from minor microorganisms is as follows: DNAs directly extracted from an environmental sample such as a soil sample are normalized using the difference in GC contents of the DNAs in the sample, and a library is then prepared (Patent Document 1). This technique cannot be used when GC contents of major microorganisms are close to those of minor microorganisms.

Examples of a compound that can be converted by the catalysis of P450s include petroleum compounds such as alkanes (linear hydrocarbons). Such alkanes are converted into diols or dicarboxylic acids by oxidizing both terminal groups of the alkanes and the diols or dicarboxylic acids are used as raw materials for producing polymers, perfumes, or medicines. Although these products are currently synthesized by petrochemical processes, the replacement of the petrochemical processes with biochemical processes is desired; hence, the biochemical oxidation of terminal groups of alkanes has been attracting much attention. Examples of P450s that can oxidize terminal groups of an alkane include a CYP52 family present in yeasts such as Candida maltosa and Candida tropicalis (Non-patent Document 4). This document discloses that Candida maltosa has four types of genes belonging to a CYP52A sub-family whose expression is induced by alkanes and the specificities of these genes are different from each other depending on the type or length of substrates such as alkanes and fatty acids. A P450, supposed to be involved in the decomposition of alkanes, originating from prokaryote has been first discovered in a strain of Acinetobacter calcoaceticus EB104 that is a bacterium that utilizes a long-chain n-alkane (Non-patent Document 5). P450s have been recently discovered in several types of bacteria, such as Alcanivorax borkumensis and Rhodococcus erythropolis, having the ability to utilize alkanes. These types of P450 form the CYP153A subfamily (Non-patent Document 6 and Dr. Nelson's homepage (http://drnelson.utmem.edu/CytchromeP450.html)). However, there is no direct evidence that these types of P450 convert terminal groups of alkanes into hydroxyl groups. It has been disclosed that the following cytochrome has an ability to oxidize alkanes with three to eight carbon atoms: P450BM-3 139-3 which is a mutant of P450 originating from Bacillus megaterium and which has been obtained by molecular evolution engineering (Non-patent Document 7). The P450 gene is of a P450/reductase-fused type and is efficiently expressed in Escherichia coli; hence, the P450 gene has been expected to be used for bioconversion processes. However, since the P450 does not convert terminal groups of alkanes but principally convert branches thereof into hydroxyl groups, the P450 has not been put to practice use. As described above, A P450 that can oxidize such alkane terminal groups is expected to be used for industrial use and have been therefore intensively investigated. However, findings about P450s are limited and the amount of information on P450s originating from bacteria is particularly small.

P450 monooxygenases are classified into two classes on the basis of the difference in electron transfer. A first class (Class I) includes P450 monooxygenases in which electrons are supplied from NAD(P)H to a P450 protein, by way of an NAD(P)H-iron-sulfur protein (ferredoxin) reductase that contains a flavin adenine dinucleotide (FAD) as a coenzyme and an iron-sulfur protein (ferredoxin) (the monooxygenases of this class are hereinafter referred to as three-component-containing P450 monooxygenases in some cases). P450 monooxygenases contained in mitochondria or bacteria usually belong to this class. A second class (Class II) includes P450 monooxygenases in which an NADPH P450 reductase containing a FAD and a flavin mononucleotide (FMN) that are coenzymes supplies a P450 protein with electrons from NADPH, in which any iron-sulfur protein is not needed (the monooxygenases of this class are hereinafter referred to as two-component-containing P450 monooxygenases in some cases). P450 monooxygenases present in microsomes from mammal liver belong to this class.

In recent years, a large number of novel genes encoding cytochromes P450 (CYP) have been discovered because of the advance in analyzing bacterial genomes. However, most of genes encoding electron transfer proteins corresponding to P450s have not been discovered. In 45 species of Streptomyces expected to be useful in synthesizing, for example, medicines, 170 or more of P450 genes have been discovered; however, only less than 20 types of electron transfer proteins corresponding to the P450 genes have been discovered (Parajuli et al., Genome analyses of Streptomyces peucetius ATCC 27952 for the identification and comparison of cytochrome P450 complement with other Streptomyces, Arch Biochem Biophys, 425: 233-241, 2004).

Some examples show that a bacterial enzyme is activated using an electron transfer protein present in the bacterium or a P450 electron transfer protein present in another bacterial strain or organism although an electron donor corresponding to the electron transfer protein is unknown. The following fact has been disclosed: some types of Streptomyces cytochromes P450 exhibit P450 monooxygenase activity in the presence of ferredoxin (putidaredoxin) and a putidaredoxin reductase originating from Pseudomonas putida ( Arisawa A, et al., Bioconversion by Escherichia coli in which actinomycete cytochrome P450 and electron transfer protein are coexpressed, Annual Meeting of Japan Society for Bioscience, Biotechnology, and Agrochemistry 2003, p. 38, 2003). Furthermore, the following fact has been disclosed: P450 (PB-1) originating from Corynebacterium sp. strain EP1 exhibits activity in the presence of an electron donor originating from spinach (Kawahara N, et al., Purification and characterization of 2-ethoxyphenol-induced cytochrome P450 from Corynebacterium sp. strain EP1., Can J Microbiol. 45: 833-839, 1999). However, the electron donors and electron transfer proteins described above cannot be necessarily applied to all types of P450; hence, an electron transfer protein compatible with P450 is sought for its use. In order to find such a compatible electron transfer protein without extraordinary efforts, the investigation of P450s needs to be focused on a specific bacterial strain or a P450 family including proteins homologous to each other. Even if an electron transfer protein functioning well can be obtained, a large amount of time must be spent optimizing gene manipulation and induction conditions (for example, whether structural genes are carried on different plasmids or the same plasmid, the order of the structural genes, the timing of inducing the structural genes) for the purpose of coexpressing genes for the electron transfer protein and P450 compatible therewith in a bacterium. In the analysis of P450 functions in vitro, reductases are separately produced, purified, and then mixed such that an optimum mixing ratio can be achieved; hence, a large amount of time and resources are spent analyzing one P450 function. Therefore, the amount of information about functions of P450 genes is small although there is a vast amount of information about the structures of the P450 genes. Hence, it is difficult to perform high-throughput selection, that is, it is difficult to efficiently select an enzyme having a catalytic function or specificity to a target substrate among various types of P450s or difficult to quickly find a substrate catalyzed by P450s.

In general, P450 has a problem in that the activity per cytochrome P450 molecule is low because the rate of an enzymatic reaction depends on the transfer of electrons from an electron donor to a cytochrome P450. The following enzyme is probably a key for solving the problem: a fused P450 monooxygenase, which is a rare example, containing a single polypeptide chain including a P450 protein and reductase linked to each other. Well-known examples are P450BM3 (Non-patent Document 8) originating from Bacillus megaterium and P450foxy originating from a mold. These cytochrome P450 monooxygenases contain a P450 protein similar to that contained in a two-component P450 monooxygenase belonging to Class II and also contain a NADPH-P450 reductase containing FAD and FMN that are coenzymes. These P450s are different from the two-component P450 monooxygenase in that the two components thereof are linked together with a linker portion disposed therebetween to form a single peptide chain. The fused P450 monooxygenase has an advantage in that the enzymatic reaction rate and activity thereof are greater than those of three- or two-component P450 monooxygenases. The reason for the advantage is that the fusion between the P450 protein and the reductase probably allows the intramolecular transfer of electrons, resulting in the formation of a three-dimensional structure due to the best interaction between the P450 protein and the reductase. Various types of artificial fused P450 monooxygenases originating from eukaryotes have been prepared in imitation of the fused P450 monooxygenase. There is, for example, an artificial fusion enzyme that includes a human liver P450 protein bound to the N-terminus thereof and an NADPH-P450 reductase, originating from yeast, rat, or P450BM3 described above, bound to the C-terminus thereof. Although P450BM3 originates from a bacterium, its P450 heme domain and its NADPH-P450 reductase domain has high homology with P450s and P450 reductases, respectively, originating from eukaryotes, belonging to Class II; hence, the P450BM3 can probably act as an electron donor. P450BM3, which is bacterial P450 as described above, is apparently different from a three-component P450 monooxygenase, originating from an ordinary bacterium, belonging to Class I.

Except for the case of P450BM3, There is only one successful example of the preparation of a fusion enzyme including a P450 reductase domain prepared using a bacterial P450 protein (a successful example of the preparation of an active fusion enzyme). In this example, components of a P450 monooxygenase that are present in the same system are only fused together (Sibbesen, O., et al. Putidaredoxin reductase-putidaredoxin-cytochrome P450cam triple fusion protein. Construction of a self-sufficient Escherichia coli catalytic system. J Biol Chem. 271: 22462-9, 1996). There is no successful example of an attempt to fuse an ordinary bacterial P450 protein and a reductase domain originating from a P450 monooxygenase component together. This is probably because the function system of bacterial P450 contains the P450 protein, ferredoxin, and the ferredoxin reductase as described above and is thus complicated. In the industrial use of bacterial P450 monooxygenases, an artificial fusion enzyme enables simple enzymatic purification or intracellular reaction; however, any technique for preparing the artificial fusion enzyme has been established yet.

A P450 monooxygenase that is not similar to the P450 monooxygenases belonging to Class I or II nor the fused P450 monooxygenase has been recently disclosed (Non-patent Document 8). This P450 monooxygenase (named P450RhF) is isolated from Rhodococcus sp. strain NCIMB 9784 and is a single peptide containing a P450 protein (a heme domain), a NADH-P450 reductase domain containing a flavin mononucleotide (FMN) that is a coenzyme, and an iron-sulfur protein domain. This P450 monooxygenase (P450RhF) is novel in that the reductase domain requires the FMN only. This is because reductases present in P450 monooxygenases of Class II or known fused P450 monooxygenases requires both FAD and FMN. Accordingly, P450RhF is exceptional among P450 monooxygenases.
Patent Document 1: PCT Japanese Translation Patent Publication No. 2000-513933
Non-patent Document 1: Urlacher et al., Appl. Microbiol. Biotechnol., 64: 317-325 (2004)
Non-patent Document 2: Thomas et al., Proc. Natl. Acad. Sci., 99: 10494-10499 (2002)
Non-patent Document 3: Okuta, A. et al., Gene, 212: 221-228 (1998)
Non-patent Document 4: Ohkuma et al., J. Biol. Chem., 273: 3948-3953 (1998)
Non-patent Document 5: Thomas et al., Biochem. Biophy. Res. Comm., 286: 652-658 (2001)
Non-patent Document 6: Beilen et al., biocat 2004 Book of Abstracts, p. 233
Non-patent Document 7: Glieder et al., Nat. Biotechol., 20: 1135-1139 (2002)
Non-patent Document 8: Roberts, G.A. et al., Identification of a new class of cytochrome P450 from a Rhodococcus sp. J. Bacteriol., 184: 3898-3908, 2002.

### Disclosure of Invention

### Problems to be Solved by the Invention

It is a first object of the present invention to provide a method for efficiently isolating various P450 genes from microbial resources present in various environments and particularly from scarce microorganisms present in such environments, to provide a novel P450 gene, to provide P450 protein synthesized using the novel P450 gene, and to provide a technique for oxidizing various organic compounds using the enzyme.

Since P450 monooxygenases are variable in substrate specificity and reaction specificity, the P450 monooxygenases have been expected to be used for various useful biocatalytic reactions. However, most of P450 proteins (CYP) have no catalytic activity by theirselves. Hence, if a novel P450 gene is discovered, a large amount of time and extraordinary efforts are required to identify a substrate catalyzed by P450 encoded by the gene and to put P450 to practical use. In order to utilize enzymatic activity (catalytic activity) that the P450 proteins must have inherently, active P450 monooxygenases must be restructured. This requires electron transfer proteins for supplying electrons to the P450 proteins. However, an electron donor corresponding to a P450 and particularly to a novel P450 cannot be readily discovered and a system containing a P450 monooxygenase has low enzymatic activity and is easily inactivated. Therefore, a system, applicable to various P450 proteins, for efficiently transferring electrons has been demanded. It is a second object of the present invention to provide a method for structuring such a system to produce an active P450 monooxygenase.

### Means for Solving the Problems

In order to achieve the first object, the inventors have performed intensive investigation. The inventors have found that amino acid sequences are well conserved in regions of the CYP153A subfamily including a plurality of P450 genes originating from microorganisms utilizing petroleum components. Furthermore, the inventors have found that fragments of novel P450 genes can be amplified from various environmental DNA samples using primers designed on the basis of the regions. The inventors have developed a technique in which a hybrid gene between an amplified P450 gene and a known P450 gene is prepared by a cassette PCR method and then analyzed for function using an Escherichia coli expression system. The inventors have further performed investigation and found that products of hybrid genes containing novel P450 sequences obtained from environments have monooxygenase activity and catalyze the oxidation of organic compounds such as alkanes (linear hydrocarbons), alkenes (linear hydrocarbons containing unsaturated bonds), cyclic hydrocarbons, alkyl aromatics, and alkenyl aromatics. In particular, the products catalyze the hydroxylation of a terminal methyl group and the epoxidation of a terminal olefin group.

In order to achieve the second object, the inventors have attempted to develop a versatile, efficient system for transferring electrons to a P450. During the attempt, the inventors have paid attention to a cytochrome P450 monooxygenase (P450Rhf) from Rhodococcus sp. strain NCIMB 9784. As disclose in Section "Background Art", P450Rhf is the fused P450 monooxygenase that is present in the form of a single peptide chain formed by the fusion of the P450 protein and the P450 reductase domain (region). The fused P450 monooxygenase is different from P450BM3 or P450foxy as described above and is novel in that the reductase domain contains the iron-sulfur protein-like component and the FMN protein-like component (Non-patent Document 1). In general, Actinobacteria such as Rhodococcus have various catalytic functions involved in the conversion of various organic components and also have complicated enzyme systems. Therefore, the use of P450Rhf, which is a novel enzyme, for general purposes has been supposed to be difficult. It is usually difficult to express a gene, involved in an enzyme system from a Rhodococcus sp. strain, in a multipurpose host such as an Escherichia coli strain. Hence, in view of common sense, it has been supposed that P450Rhf cannot be used for the purpose of the inventors, that is, the development of the versatile, efficient system for transferring electrons to a P450. The inventors have investigated if the reductase domain of P450Rhf can be used to transfer electrons to various bacterial P450 proteins and if the reductase domain can be allowed to serve as a fusion protein for inducing intramolecular electron transfer. As a result, this investigation showed that a P450 monooxygenase which has catalytic activity and which is hardly inactivated can be prepared by fusing the P450Rhf reductase domain and the C-terminus of a bacterial P450 protein with an appropriate linker region disposed therebetween.

In particular, the inventors have prepared a single polypeptide gene in such a manner that a gene encoding a bacterial cytochrome P450 protein (CYP) is linked to a gene encoding the reductase domain of P450Rhf from Rhodococcus sp. strain NCIMB 9784, thereby structuring a system for expressing an artificial fusion enzyme gene encoding an artificial fusion enzyme having monooxygenase activity originating from the P450 and reducing ability originating from the P450Rhf reductase domain. Furthermore, the inventors have structured such an expression system using different bacterial P450 genes, thereby allowing an Escherichia coli strain to produce an artificial fusion enzyme. The inventors have confirmed that the produced artificial fusion enzyme, which is a single molecule, has electron transfer ability and substrate oxidation ability and that the expression system is applicable to various P450 genes. Furthermore, the inventors have discovered that if the expression system is used, the biocatalytic activity of a P450 can be determined by a simple bioconversion reaction using culture of Escherichia coli.

The present invention has been made on the basis of the above findings.

The present invention is as specified in Items (1) to (23) below.
(1) An oligonucleotide, having substantially a primer or probe function, for isolating a P450 gene fragment contains a nucleotide sequence encoding part or all of the amino acid sequence set forth in SEQ ID NO: 51 or 52 or a nucleotide sequence complementary thereto.
(2) An oligonucleotide, having substantially a primer or probe function, for isolating a P450 gene fragment contains the nucleotide sequence set forth in any one of SEQ ID NOS: 53 to 56 or part or all of a nucleotide sequence complementary thereto.
(3) A method for isolating a P450 gene fragment includes a step of using the oligonucleotide according to Item (1) or (2) as at least one primer.
(4) A method for isolating a P450 gene fragment includes a step of extracting a nucleic acid from a sample and a step of performing nucleic acid amplification using the nucleic acid and the oligonucleotide according to Item (1) or (2) as a template and a primer, respectively.
(5) A method for preparing a hybrid P450 gene includes a step of isolating a P450 gene fragment using the oligonucleotide according to Item (1) or (2) as at least one primer and a step of adding a 5'-terminal region and 3'-terminal region of a known P450 gene to the 5'-terminus and 3'-terminus, respectively, of the isolated P450 gene.
(6) In the method according to Item (5), the known P450 gene encodes the amino acid sequence set forth in SEQ ID NO: 57.
(7) A kit for isolating a P450 gene includes:
   (a) the oligonucleotide according to Item (1) or (2);
   (b) a DNA fragment containing a 5'-terminal region of a known P450 gene; and
   (c) a DNA fragment containing a 3'-terminal region of a known P450 gene.
(8) A gene encodes the protein specified in any one of Items (a) and (b) below:
   (a) a protein containing the amino acid sequence set forth in any one of SEQ ID NOS: 1 to 25; and
   (b) a protein, serving as P450, containing an amino acid sequence prepared by removing one or more amino acid residues from the amino acid sequence set forth in any one of SEQ ID NOS: 1 to 25, replacing one or more amino acid residues of this amino acid sequence with other residues, or adding one or more amino acid residues to this amino acid sequence.
(9) A gene encodes a protein, serving as P450, containing the nucleotide sequence set forth in any one of SEQ ID NOS: 26 to 50.
(10) An expression vector containing the gene according to Item (8) or (9).
(11) A protein is specified in any one of Items (a) and (b) below:
   (a) a protein containing the amino acid sequence set forth in any one of SEQ ID NOS: 1 to 25; and
   (b) a protein, serving as P450, containing an amino acid sequence prepared by removing one or more amino acid residues from the amino acid sequence set forth in any one of SEQ ID NOS: 1 to 25, replacing one or more amino acid residues of this amino acid sequence with other residues, or adding one or more amino acid residues to this amino acid sequence.
(12) A method for producing a protein serving as P450 includes a step of transforming the expression vector according to Item (10) into a host cell and then culturing the host cell and a step of recovering the protein according to Item (11) from the cultured cells.
(13) A method for producing an oxidized compound includes a step of subjecting a substrate to a reaction in the presence of the protein according to Item (11) to produce an oxidized compound.
(14) A fused cytochrome P450 monooxygenase contains a peptide which is linked to the C-terminus of a P450 protein with a linker portion disposed therebetween and which has the same function as that of a reductase domain contained in a cytochrome P450 monooxygenase originating from Rhodococcus sp. strain NCIMB 9784.
(15) In the fused cytochrome P450 monooxygenase according to Item (14), the peptide is (a) one containing the amino acid sequence set forth in SEQ ID NO: 70; (b) one containing an amino acid sequence prepared by adding one or more amino acid residues to the amino acid sequence set forth in SEQ ID NO: 70; removing one or more amino acid residues from this amino acid sequence, or replacing one or more amino acid residues of this amino acid sequence with other residues; or (c) one encoded by a DNA hybridized with a DNA containing the nucleotide sequence set forth in SEQ ID NO: 69 or a DNA complementary to this DNA under stringent conditions.
(16) In the fused cytochrome P450 monooxygenase according to Item (14) or (15), the linker portion is (d) a peptide containing the amino acid sequence set forth in SEQ ID NO: 68; (e) a peptide containing an amino acid sequence prepared by adding one or more amino acid residues to the amino acid sequence set forth in SEQ ID NO: 68; removing one or more amino acid residues from this amino acid sequence, or replacing one or more amino acid residues of this amino acid sequence with other residues; or (f) a peptide encoded by a DNA hybridized with a DNA containing the nucleotide sequence set forth in SEQ ID NO: 67 or a DNA complementary to this DNA under stringent conditions.
(17) In the fused cytochrome P450 monooxygenase according to any one of Items (14) to (16), the P450 protein originates from a bacterium.
(18) In the fused cytochrome P450 monooxygenase according to any one of Items (14) to (16), the P450 protein is identical to the protein according to Item 11 or a protein containing the amino acid sequence set forth in SEQ ID NO: 85.
(19) A DNA encodes the fused cytochrome P450 monooxygenase according to any one of Items (14) to (18).
(20) A microorganism contains the DNA according to Item (19).
(21) A method for producing a fused cytochrome P450 monooxygenase includes a step of culturing the microorganism according to Claim 20 and a step of recovering a fused cytochrome P450 monooxygenase from the cultured microorganisms or cells thereof.
(22) A method for producing an oxidized compound includes a step of subjecting a substrate to a reaction in the presence of the fused cytochrome P450 monooxygenase according to any one of Claims 14 to 18 to produce an oxidized compound.
(23) In the method according to Item (22), the substrate is n-hexane, n-heptane, n-octane, n-decane, 1-octene, cyclohexane, n-butylbenzene, 4-phenyl-1-butene, or 2-n-butylbenzofran and the oxidized compound is 1-hexanol, 1-heptanol, 1-octanol, 1-decanol, 1,2-epoxyoctane, cyclohexanol, 4-phenyl-1-butanol, 2-phenethyloxirane, or 4-benzofran-2-yl-butane-1-ol.
(24) A gene encodes the protein specified in any one of Items (a) and (b) below:
   (a) a protein containing the amino acid sequence set forth in SEQ ID NO: 85; and
   (b) a protein, serving as P450, containing an amino acid sequence prepared by removing one or more amino acid residues from the amino acid sequence set forth in SEQ ID NO:85, replacing one or more amino acid residues of this amino acid sequence with other residues, or adding one or more amino acid residues to this amino acid sequence.
(25) A gene encodes a protein, serving as P450, containing the nucleotide sequence set forth in SEQ ID NO: 86.

### Advantages

The present invention provides a method for efficiently isolating a novel P450 gene from a sample, such as an environmental sample, containing various microbial nucleic acids. Cytochromes P450 synthesized from the P450 genes obtained by the method can be used to produce various low-molecular-weight organic compounds.

Fused P450 monooxygenases according to the present invention are prepared from known P450 proteins and unknown P450 proteins. The fused P450 monooxygenases is of a single component type which has electron transfer ability and substrate oxidation ability. Hence, an experiment for reconstructing an electron transfer protein and a reaction system is not necessary although such an experiment had been necessary to achieve P450 monooxygenase activity. This leads to practical applications such as the high throughput screening of useful active biocatalysts, the simple purification of a useful P450 enzyme, a bioconversion process for producing a useful substance by use of a microorganism containing a fusion enzyme gene, and the oxidative removal of harmful substances in industrial waste water.

### Brief Description of the Drawings

[Fig. 1] Fig. 1 is an illustration showing a phylogenetic tree based on the homology between amino acid sequences (SEQ ID NOS: 1 to 25) of obtained novel P450s and amino acid sequences of known P450s.
[Fig. 2] Fig. 2 includes graphs showing changes in the absorption spectra of Crude Extract Solution A (Fig. 2A), Crude Extract Solution B (Fig. 2B), and Crude Extract Solution C (Fig. 2C) treated with carbon monoxide under reductive conditions.
[Fig. 3] Fig. 3 includes charts obtained by analyzing reaction solutions obtained by subjecting octane and 1-decene to enzyme reactions using Crude Enzyme Solutions B and D.
[Fig. 4] Fig. 4 is an illustration showing the structure of an expression plasmid for preparing fused P450.
[Fig. 5] Fig. 5 is a graph showing the comparison between the productions of 1-alcohols produced by oxidizing n-alkanes with six to eight carbon atoms.
[Fig. 6] Fig. 6 includes charts obtained by analyzing inducers by gas chromatography-mass spectrometry, the inducers being converted from reaction products prepared by subjecting n-butylbenzene to reactions using Bacterial Suspension H5, H9, or Alk or control Bacterial Suspension Non.
[Fig. 7] Fig. 7 includes charts obtained by analyzing reaction products by gas chromatography-mass spectrometry, the reaction products being obtained by subjecting 4-phenyl-1-butene to reactions using Bacterial Suspension H5, H9, or Alk or control Bacterial Suspension Non.
[Fig. 8] Fig. 8 includes graphs showing optical properties of artificial fused P450cam (Fig. 8A) and non-fused P450cam (Fig. 8B).
[Fig. 9] Fig. 9 is a graph showing the conversion of camphor to 5-hydroxycamphor by use of a strain of Escherichia coli producing fused or non-fused P450cam.
[Fig. 10] Fig. 10 is a graph showing the conversion of n-octane to 1-octanol by use of a strain of Escherichia coli producing fused or non-fused P450alk.
[Fig. 11] Fig. 11 is a graph showing the conversion of 4-hydroxybenzoic acid to 3,4-dihydroxybenzoic acid by use of a strain of Escherichia coli producing fused or non-fused P450bzo.

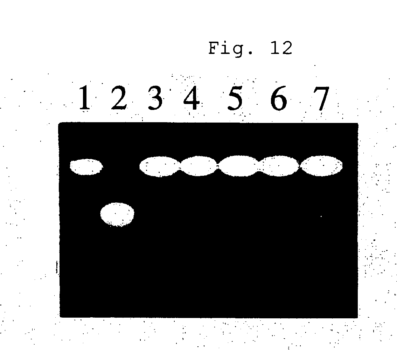

[Fig. 12] Fig. 12 is a chart showing the conversion of 7-ethoxycoumarin to 7-hydroxycoumarin by use of a strain of Escherichia coli producing fused or non-fused P450SU-1 or fused or non-fused P450SU-2, wherein Lane Number 1 corresponds to 7-ethoxycoumarin, Lane Number 2 corresponds to 7-hydroxycoumarin, Lane Number 3 corresponds to a strain containing no P450, Lane Number 4 corresponds to a strain containing pETP450SU-1, Lane Number 5 corresponds to a strain containing pSU-1RED, Lane Number 6 corresponds to a strain containing pETP450SU-2, and Lane Number 7 corresponds to a strain containing pSU-2RED.

### Best Mode for Carrying Out the Invention

### 1. P450 Monooxygenase

A P450 monooxygenase is a complex enzyme that requires an electron transfer protein in addition to a cytochrome P450 protein (CYP). In order that P450 monooxygenases are active, P450 monooxygenases present in bacteria or mitochondria require a ferredoxin reductase and ferredoxin that are proteins for transferring electrons from NAD(P)H and P450 monooxygenases present in microsomes from mammal liver require an NADPH-P450 reductase. The primary structure of the P450 monooxygenase is characterized in that a common sequence of FXXGXR/HXCXG is 50 to 60 residues apart from the C-terminus thereof. This sequence is referred to as a heme-binding region, which is characteristic of the P450 monooxygenase. When this region is present in an amino acid sequence predicted from a nucleotide sequence, a DNA thereof is defined as a gene that encodes the P450 monooxygenase (Omura et al., Molecular Biology of P450, Kodansha Scientific, 2003). P450 genes are classified according to the degree of similarity between amino acid sequences and the latest information about the classification is available on Dr. Nelson's homepage (http://drnelson.utmem.edu/CytchromeP450.html). According to the nomenclature authorized therein, a gene that belongs to the P450 superfamily has the prefix CYP followed by a family number, a subfamily number, and a gene number. Genes with amino acid sequences of which 40% or more are classified as the same family and genes with amino acid sequences of which 55% or more are classified as the same subfamily in principle.

As used herein, the term "monooxygenase activity" means the ability to catalyze a reaction in which one mole of water is produced by reducing one atom of an oxygen molecule and a low-molecular-weight organic compound is converted into an oxidation product by introducing the other one into the compound. The term "P450 monooxygenase" means a protein that has a difference spectrum (CO difference spectrum) with a maximum at 450 nm determined by applying carbon monoxide to the protein in a reduced state and also has monooxygenase activity.

### 2. P450 Monooxygenase CYP153 Family

A CYP153A1 monooxygenase belonging to the P450 monooxygenase CYP153 family has been first discovered in an Acinetobacter calcoaceticus EB104 strain (Non-patent Document 5). Thereafter, P450 monooxygenases belonging to the CYP153 family have been discovered in several species of microorganisms with the ability to utilize alkanes (Dr. Nelson's homepage (http://drnelson.utmem.edu/CytchromeP450.html). There is a report on a function of a P450 monooxygenase belonging to the CYP153 family (Non-patent Document 6). The report discloses that a CYP153A1 gene is expressed in a Pseudomonas putida host with no ability to utilize an alkane (octane) and the host is grown in a medium containing octane. From the observation of the host, the report suggests that the host has a P450 monooxygenase introducing hydroxyl groups into terminal groups of octane molecules. There is no report providing direct evidence that any P450 monooxygenase as well as the P450 monooxygenase belonging to the CYP153 family has the ability to introduce hydroxyl groups into terminal groups of alkanes. The inventors are the first to report a new P450 monooxygenase with that ability. Ordinary microbial P450 monooxygenases probably require a ferredoxin reductase and ferredoxin that are electron transfer proteins for catalyzing oxidation.

### 3. Cassette PCR Method

### (1) Preparation of oligonucleotides for isolating P450 gene fragment

Oligonucleotides (hereinafter referred to as "present oligonucleotides") for isolating a fragment of a P450 gene according to the present invention contains a nucleotide sequence encoding part or all of the amino acid sequence set forth in SEQ ID NO: 51 or 52 or a nucleotide sequence complementary thereto and has substantially a primer function. Since the present oligonucleotides are used as primers, such a P450 gene fragment can be isolated. As used herein, the term "oligonucleotide" means a deoxyribonucleotide or a ribonucleotide and the term "nucleic acid" means DNA or RNA.

The present oligonucleotides are designed on the basis of amino acid sequences each present in CYP153A1 (accession no. AJ311718), CYP153A2 (accession no. AE005680), or CYP153A13a (SEQ ID NO: 57) registered in Dr. Nelson's homepage (http://drnelson.utmem.edu/CytchromeP450.html). That is, the present oligonucleotides are designed on the basis of P450-conserved regions predicted from the alignment of these sequences.

The present oligonucleotides are designed to have substantially a primer function. As used herein, the clause "an oligonucleotide has substantially a primer function" means that an oligonucleotide meets requirements for specific annealing or hybridizing, that is, an oligonucleotide has a length and nucleotide composition (melting temperature) sufficient for specific annealing or hybridizing. This means that the present oligonucleotides are different from an oligonucleotide which has a small sequence length or an unsuitable nucleotide composition and which therefore usually promotes nonspecific annealing. The present oligonucleotides preferably have a length of ten bases or more, more preferably 16 bases or more, and most preferably 18 bases or more. Preferable examples of the present oligonucleotides include oligonucleotides containing the base sequences set forth in SEQ ID NOS: 53 to 56.

The present oligonucleotides may contain assembly sequences, linked to their 5'-terminuses, for preparing a hybrid gene described below. The assembly sequences are defined as sequences that can be annealed with a known P450 gene fragment. P450 gene fragments amplified using the present oligonucleotides as a forward primer or a reverse primer only have a sequence corresponding to a region sandwiched between the forward primer and the reverse primer. That is, the amplified P450 gene fragments lack a 5'-terminal sequence containing an initiation codon and a 3'-terminal sequence containing a termination codon. These lacking sequences can be supplemented in such a manner that sequences are annealed with the 5' - or 3'-terminal fragment of a known P450 gene and then subjected to an elongation reaction. The sequences annealed with the 5'- or 3'-terminal fragment of the known P450 gene correspond to the assembly sequences. When one of the present oligonucleotides is used as the forward primer, one of the assembly sequences is usually the 3'-terminal sequence of a known 5'-terminal fragment. When the other one of the present oligonucleotides is used as the reverse primer, the other one of the assembly sequences is usually the 5'-terminal sequence of a known 3'-terminal fragment. Examples of the present oligonucleotides include oligonucleotides containing the assembly sequences.

The assembly sequences may have such a length that the assembly sequences can be annealed with known 5'- or 3'-terminal fragments and the amplification of nucleic acids is inhibited. The assembly sequences preferably have a length of five to 50 bases and more preferably ten to 20 bases.

The present oligonucleotides can be synthesized by a method, such as a phosphite method or a phosphotriester method, known to those skilled in the art.

### (2) Isolation of fragments of novel P450 gene

Fragments of a novel P450 gene can be isolated using the oligonucleotides specified in Subsection "(1) Preparation of oligonucleotides for isolating P450 gene fragment". In usual, such novel P450 gene fragments can be isolated by an amplification reaction using the present oligonucleotides as primers (this method is hereinafter referred to as "present isolation method").

In the present isolation method, any sample containing nucleic acid originating from a prokaryote can be used to isolate the novel P450 gene fragments. Examples of such a sample include artificially cultured microorganisms and environmental samples containing a variety of unknown microorganisms. Examples of such environmental samples include brine, freshwater, and soil. In the present isolation method, a nucleic acid is extracted from the sample in usual. The extracted nucleic acid may be DNA or RNA. DNA or RNA can be extracted by a method known to those skilled in the art. In the extraction of DNA, the following method can be used: a method including phenol extraction and ethanol precipitation, a method using glass beads, or another method. In the extraction of RNA, the following method can be used: a guanidine-cesium chloride ultra-centrifugal separation method, a hot phenol method, an acid-guanidine thiocyanate-phenol-chloroform (AGPC) method, or another method. When the sample is brine or freshwater, the sample is preferably concentrated before nucleic acids are extracted therefrom. The sample can be concentrated by filtering the sample with a filter with an appropriate pore size. A material for forming the filter is not particularly limited and any material used to prepare ordinary filters may be used. Examples of such a material include cellulose acetate, nitrocellulose, cellulose-mixed esters, polyvinylidene difluoride, polytetrafluoroethylene, polycarbonates, polypropylene, and silver. The following method has been disclosed: a method in which a carrier bearing a culture medium is placed in water and nucleic acids from microorganisms attached to the carrier are efficiently recovered (Japanese Unexamined Patent Application Publication No. 2003-334064). After cells are lysed with a surfactant such as SDS as required, nucleic acids can be extracted from the sample obtained as described above by any one of the above methods.

When the sample is soil, nucleic acids can be extracted by a method known to those skilled in the art. Examples of a method for extracting nucleic acids from soil include the Zhou et al. method (Zhou et al., Appl. Environ. Microbiol., 62: 316-322 (1996)) and the Griffiths et al. method (Griffiths et al., Appl. Environ. Microbiol., 66: 5488-5491 (2000)).

The P450 gene fragments can be isolated from the nucleic acid obtained as described above using the oligonucleotides specified in Subsection "(1) Preparation of oligonucleotides for isolating P450 gene fragment". The P450 gene fragments can be specifically amplified by a nucleic acid amplification reaction in such a manner that the nucleic acid obtained as described above is used as a template and the oligonucleotides according to the present invention are used as primers. In usual, one of the present oligonucleotides that is designed on the basis of the amino acid sequence set forth in SEQ ID NO: 51 is used as a forward primer and the other one designed on the basis of the amino acid sequence set forth in SEQ ID NO: 52 is used as a reverse primer. The present oligonucleotides used in such a nucleic acid amplification reaction may be degenerated primers. A method for performing the nucleic acid amplification reaction is not particularly limited and any method using the present oligonucleotides as primers can be used. Examples of the most preferable method include a known method using a polymerase chain reaction (PCR) technique. The amplification reaction is continued until an amplified product is detected. The PCR technique is used to synthesize a nucleotide sequence between a pair of primers using a DNA polymerase. According to the PCR technique, amplification fragments can be exponentially amplified by repeating a cycle of denaturation, annealing, and synthesis. Those skilled in the art can determine optimum conditions of PCR. An RT-PCR technique is as follows: cDNA is prepared by a reverse transcriptase reaction using RNA as a template and PCR is performed using a pair of primers and the prepared cDNA as a template. In the case that the present oligonucleotides are used, those skilled in the art can determine conditions for specifically amplifying the P450 gene on the basis of the length and nucleotide composition of the present oligonucleotides. Amplified fragments of the P450 gene can be obtained in such a manner that PCR products are fractionated by agarose gel electrophoresis or the like, amplified bands are excised, and DNA is then extracted.

### (3) Method for preparing hybrid P450 gene

The P450 gene fragments amplified by the method specified in Subsection "(2) Isolation of fragments of novel P450 gene" each contain only a sequence corresponding to a domain sandwiched between regions annealed with the present oligonucleotides. That is, the P450 gene fragments contain no 5'-terminal region containing an initiation codon or 3'-terminal region containing a termination codon. Therefore, The P450 gene fragments amplified by the method specified in Subsection (2) have no function for forming the P450 gene in some cases. In order to solve this problem and in order to obtain DNA fragments useful in preparing the P450 gene, both terminuses of each P450 gene fragment amplified by the method specified in Subsection (2) may be supplemented with sequences contained in a known P450 gene. Any P450 gene originating from a known bacterium can be used to supplement the terminuses thereof and a known P450 gene containing nucleotide sequences encoding the amino acid sequences set forth in SEQ ID NOS: 51 and 52 is preferably used. Examples of such a known P450 gene include genes belonging to CYP153A of the 450 superfamily registered in Dr. Nelson's homepage (http://drnelson.utmem.edu/CytchromeP450.html).

A hybrid P450 gene can be prepared by supplementing both terminuses of the P450 gene fragments amplified by the method specified in Subsection (2) with sequences contained in the known P450 gene. The hybrid P450 gene contains a nucleotide sequence, contained in an unknown P450 gene, located at a center region of the hybrid P450 gene and also contains nucleotide sequences, contained in the known P450 gene, located at both end regions thereof. A cytochrome P450 can be produced by introducing the hybrid P450 gene into a host. The hybrid P450 gene can be prepared in such a manner that the P450 gene fragments amplified by the method specified in Subsection (2) are annealed with the 5'-terminal region (corresponding to a region from an initiation codon to a portion annealed with the forward primer used in the amplification method specified in Subsection (2) and hereinafter referred to as "5-arm fragment") of the known P450 gene or the 3'-terminal region (corresponding to a region from a portion annealed with the reverse primer used in the amplification method specified in Subsection (2) to a termination codon and hereinafter referred to as "3-arm fragment") thereof and then subjected to an elongation reaction.

Primers used to prepare the hybrid P450 gene may contain the assembly sequences described in Subsection "(1) Preparation of oligonucleotides for isolating P450 gene fragment". The use of such primers containing the assembly sequence allows the amplified P450 gene fragments to be efficiently annealed with the 5- or 3-arm fragment. The 5-and 3-arm fragments can be prepared by a nucleic acid amplification reaction using DNA, containing a gene similar to the known P450 gene for supplementing both terminal regions, as a template. The primer can be designed and synthesized by a technique known to those skilled in the art. Conditions of the nucleic acid amplification reaction can be determined on the basis of data known to those skilled in the art. The hybrid P450 gene, prepared in such a manner that the P450 gene fragments amplified by the method specified in Subsection (2) are annealed with the 5-arm fragment or the 3-arm fragment and then subjected to an elongation reaction, can be amplified by a nucleic acid amplification reaction. The 5- and 3-arm fragments can be directly used as primers for this amplification reaction and oligonucleotide primers containing the following sequences are preferably used: a nucleotide sequence corresponding to a region containing an initiation codon identical to that contained in the 5-arm fragment and a nucleotide sequence corresponding to a region containing a termination codon identical to that contained in the 3-arm fragment. These primers can be designed and synthesized by a technique known to those skilled in the art. Conditions of this amplification reaction can be determined on the basis of data known to those skilled in the art. The hybrid P450 gene can be isolated in such a manner that amplified nucleic acid fragments are fractionated by agarose gel electrophoresis or the like, amplified bands are excised, and DNA is then extracted.

Whether the P450 gene fragments prepared by the method specified in Subsection (2) and the hybrid P450 gene prepared by the above method are genes encoding P450 can be determined by checking whether an amino acid sequence predicted from a nucleotide sequence has a common sequence of FXXGXR/HXCXG that is 50 to 60 residues apart from the C terminus. Alternatively, the above matter can be determined in such a manner that an expression vector containing the obtained nucleic acid fragments is introduced into an appropriate host and cells of the cultured host or a crude solution prepared by disrupting the cells and then subjecting the disrupted cells to extraction is measured for CO difference spectrum and monooxygenase activity.

The preparation of the expression vector, the selection of the host, and the introduction of the expression vector into the host are disclosed in various laboratory manuals (for example, Sambrook, J., Russel, D. W., Molecular Cloning, A Laboratory Manual, 3rd Edition, CSHL Press, 2001) and thus may be performed by a genetic engineering technique disclosed therein.

Examples of the expression vector introduced into the host include phages, phagemid vectors, and plasmid vectors. The host is not particularly limited to eukaryotic cells or prokaryotic cells and any host in which the expression vector can survive may be used. Examples of the host include animal cells, yeast cells, actinomycetes, and bacterial cells. Preferable examples of the bacterial cells include Escherichia coli cells and Bacillus subtilis cells. The expression vector needs to have an expression control region, such as a promoter, located upstream of the gene fragments or hybrid gene inserted therein. When the host is, for example, Escherichia coli, the expression control region is a lac promoter or the like. The host containing the expression vector is cultured in a culture medium suitable for the host and cells of the cultured host or a crude solution prepared by disrupting the cells and then subjecting the disrupted cells to extraction is measured for CO difference spectrum and monooxygenase activity. The measurement of CO difference spectrum can be performed in such a manner that the crude solution is treated with sodium dithinaite (Na₂S₂O₄), carbon monoxide is applied to the resulting solution, and the absorption spectrum of the resulting solution is measured, whereby a difference spectrum with a maximum at 450 nm is obtained. The measurement of monooxygenase activity can be performed in such a manner that a crude solution prepared by subjecting cells of the cultured host to extraction is allowed to react with a substrate and whether the substrate is oxidized is checked, the host being cultured in the presence of a low-molecular-weight organic compound under conditions that an expression vector containing the P450 gene fragments or the hybrid gene and an electron transfer protein is expressed or cultured such that the expression vector is expressed.

The P450 gene fragments or the hybrid P450 gene can be obtained by extracting DNA from the clone cells cultured by the above method.

### 4. P450 Gene-isolating Kit

A P450 gene-isolating kit (hereinafter referred to as "present kit") according to the present invention is used to isolate the hybrid P450 gene specified in Section "3. Cassette PCR Method". The present kit contains the oligonucleotides, 5-arm fragment, and 3-arm fragment specified in Section "3. Cassette PCR Method".

### (1) Primers for first stage of nucleic acid amplification reaction

As described in Section "3. Cassette PCR Method", in order to amplify the hybrid P450 gene, the nucleic acid amplification reaction must be performed in two stages. In the first stage of the nucleic acid amplification reaction, the present oligonucleotides specified in Subsection "(1) Preparation of oligonucleotides for isolating P450 gene fragment" are used as a forward primer or a reverse primer. In particular, one of the present oligonucleotides that contains a nucleotide sequence encoding part or all of the amino acid sequence set forth in SEQ ID NO: 51 is used as the forward primer and the other one containing a sequence complementary to a nucleotide sequence encoding part or all of the amino acid sequence set forth in SEQ ID NO: 52 is used as the reverse primer. The present kit contains at least one pair of the forward and reverse primers. The forward and reverse primers may be degenerated primers.

The forward and reverse primers contained in the present kit may contain assembly sequences.

### (2) 5-arm Fragment and 3-arm Fragment

The present kit contains the 5- and 3-arm fragments specified in Section "3. Cassette PCR Method". The 5-arm fragment is defined as a nucleic acid fragment containing a region, similar to that contained in a known P450 gene, from an initiation codon to a portion encoding the amino acid sequence set forth in SEQ ID NO: 51. The 3-arm fragment is defined as a nucleic acid fragment containing a region, similar to that contained in the known P450 gene, from a portion encoding the amino acid sequence set forth in SEQ ID NO: 52 to a termination codon. If the primers specified in Subsection "(1) Preparation of oligonucleotides for isolating P450 gene fragment" contain the assembly sequences, the 5- and 3-arm fragments need not contain the portion encoding the amino acid sequence set forth in SEQ ID NO: 51 or 52. The known P450 gene is used to prepare the 5- and 3-arm fragments and any bacterial P450 gene may be used for such a purpose. The known P450 gene preferably contains nucleotide sequences encoding the amino acid sequences set forth in SEQ ID NOS: 51 and 52. The 5- and 3-arm fragments may be a single- or double-stranded DNA.

### (3) Other Components

The present kit may contain a buffer for the nucleic acid amplification reaction, a dNTP mixture, enzymes (DNA polymerases and other enzymes), and the like. The present kit may contain a forward primer designed on the basis of a region containing an initiation codon of the 5-arm fragment and a reverse primer designed on the basis of a region containing a termination codon of the 3-arm fragment.

### 5. Novel P450 gene

A novel P450 gene (hereinafter referred to as "present P450 gene") according to the present invention encodes the protein specified in Item (a) or (b) below.
(a) A protein containing the amino acid sequence set forth in any one of SEQ ID NOS: 1 to 25 and 85.
(b) A protein, serving as P450, containing an amino acid sequence prepared by removing one or more amino acid residues from the amino acid sequence set forth in any one of SEQ ID NOS: 1 to 25 and 85, replacing one or more amino acid residues of this amino acid sequence with other residues, or adding one or more amino acid residues to this amino acid sequence.

The protein containing the amino acid sequence set forth in any one of SEQ ID NOS: 1 to 25 other than SEQ ID NO: 85 is produced by a hybrid P450 gene prepared on the basis of DNA, extracted from an environmental sample, by the method specified in Subsection "(3) Method for preparing hybrid P450 gene" of Section "3. Cassette PCR Method". The protein containing the amino acid sequence set forth in SEQ ID NO: 85 is encoded by a novel gene (SEQ ID NO: 86) isolated from a Nocardiaceae Hou Blue strain which is gram-positive. Examples of a gene encoding the protein specified in Item (a) include a gene represented by the nucleotide sequence set forth in any one of SEQ ID NOS: 26 to 50 and 86.

The protein specified in Item (b) is one that is prepared by introducing a small number of amino acid mutations into the protein specified in Item (a) such that the protein does not lose any P450 function. Examples of the amino acid mutations include natural amino acid mutations and artificial amino acid mutations. Examples of a technique for introducing such artificial amino acid mutations include a procedure for the construction of site-specific mutations (Nucleic acids res. 10, 6487-6500, 1982) and the technique is not limited to the procedure. The number of the amino acid mutations is not particularly limited and any number of the amino acid mutations may be used as long as this protein has monooxygenase activity. The number of the amino acid mutations is preferably 20 or less and more preferably 10 or less.

The present P450 gene can be prepared by the method specified in Subsection "(3) Method for preparing hybrid P450 gene" of Section "3. Cassette PCR Method" using a CYP153A13a gene cloned from Alcanivorax borkumensis SK2 encoding the amino acid sequence set forth in SEQ ID NO: 57.

The present P450 gene can be prepared by a procedure below. A central sequence originating from DNA extracted from an environmental sample is synthesized by a known DNA-synthesizing method. A 5-arm fragment and a 3-arm fragment are amplified by a nucleic acid amplification reaction using a set of primers represented by the nucleotide sequences set forth in SEQ ID NOS: 54 and 61 or a set of primers represented by the nucleotide sequences set forth in SEQ ID NOS: 56 and 62 in such a manner that DNA extracted from Alcanivorax borkumensis SK2 is used as a template. An elongation reaction is performed in such a manner that the synthesized central sequence is annealed with the 5- and 3-arm fragments. The present P450 gene can be amplified by a nucleic acid amplification reaction using a set of primers represented by the nucleotide sequences set forth in SEQ ID NOS: 61 and 62. The amplified present P450 genes can be then obtained in such a manner that reaction products are fractionated by agarose gel electrophoresis or the like and a fractionated band of about 1.4 Kbp is excised.

DNA fragments obtained by the above procedure can be determined to be identical to the present P450 gene in such a manner that the nucleotide sequences thereof are determined by a known method such as the dideoxy chain termination method or the Maxam-Gilbert method.

The present P450 gene can be prepared on the basis of nucleotide sequence data by chemical synthesis other than the above method.

### 6. Novel P450

Novel P450 according to the present invention is a protein specified in Item (a) or (b) below.
(a) A protein containing the amino acid sequence set forth in any one of SEQ ID NOS: 1 to 25 and 85.
(b) A protein, serving as P450, containing an amino acid sequence prepared by removing one or more amino acid residues from the amino acid sequence set forth in any one of SEQ ID NOS: 1 to 25 and 85, replacing one or more amino acid residues of this amino acid sequence with other residues, or adding one or more amino acid residues to this amino acid sequence.

The novel P450 can be prepared in such a manner that an expression vector containing the P450 gene specified in Section "5. Novel P450 gene" is introduced into a host cell and the resulting cell is cultured in a culture medium. The expression vector can be prepared and then introduced into the host cells by the known methods as described above. The novel P450 prepared as described above can be purified in such a manner that the cultured host cells are disrupted and then treated by a method, such as ion-exchange chromatography, partition chromatography, gel permeation chromatography, or affinity chromatography, widely used in the purification and/or production of protein. A P450 fraction treated by this method can be determined depending on the presence of CO difference spectrum.

### 7. Production of oxidized compound using P450

The present P450 serves as a catalyst and thus an oxidized compound can be produced using the present P450 (hereinafter referred to as "present production method"). Examples of the present production method include a method for producing the oxidized compound using a host cell into which an expression vector containing the present P450 gene has been introduced and a method for producing the oxidized compound using the purified present P450.

In order to allow the present P450 to catalyze oxidation, these methods usually require an electron transfer protein. For the use of a bacterium, a ferredoxin or a ferredoxin reductase is usually used as the electron transfer protein. For the production of the oxidized compound using the host cell, it is preferable that a gene encoding the electron transfer protein be co-expressed or a fused protein gene encoding the electron transfer protein and the present P450 be co-expressed. Alternatively, an electron transfer protein originating from the host cell may be used. For the production of the oxidized compound using the purified present P450, that electron transfer protein is preferably used in combination with NAD(P)H.

Any compound catalyzed by the present P450 can be used as a substrate in the present production method. According to the present production method, for example, an alcohol can be produced from an alkane and an epoxide can be produced from an alkene.

For the use of the purified enzyme, the present production method may be performed in a solution or in such a manner that the purified present P450 is immobilized on an appropriate carrier and then contacted with the substrate. The carrier for supporting the present P450 is not particularly limited. Examples of the carrier include inorganic carriers such as glass beads and silica gel beads; organic carriers made of cellulose, chitosan, Sepharose, dextran, polyvinyl alcohol, an ethylene-vinyl acetate copolymer, polyacrylamide, a polyacrylic acid, a polymethacrylic acid, polymethylmethacrylate, cross-linked polyvinyl alcohol, cross-linked polyacrylate, cross-linked polyacrylamide, cross-linked polystyrene, cross-linked cellulose, cross-linked agarose, or cross-linked dextran; and composite carriers made of these materials. The carrier preferably has a functional group, disposed thereon, for immobilizing the present P450. Examples of the functional group include a hydroxyl group, an amino group, an aldehyde group, a carboxyl group, a thiol group, a silanol group, an epoxy group, an amide group, a halogen group, a succinylimide group, and an acid anhydride group.

In the immobilization of the present P450 on the carrier, a hydrophilic spacer is preferably placed between the present P450 and the carrier such that the steric hindrance between the protein and the carrier is reduced, the efficiency of adsorption is thereby increased, and nonspecific adsorption is prevented. Examples of the hydrophilic spacer include organic molecules having terminal substituents such as carboxyl groups, amino groups, aldehyde groups, or epoxy groups.

### 8. Novel Fused Cytochrome P450 Monooxygenase

A novel fused cytochrome P450 monooxygenase has been recently disclosed (Roberts, G.A., et al, Identification of a new class of cytochrome P450 from a Rhodococcus sp. J. Bacteriol. 184: 3898-3908, 2002). This is a P450 monooxygenase (named "P450RhF") isolated from Rhodococcus sp. strain NCIMB 9784 and is a single peptide chain containing a P450 protein (heme domain), an NADH-P450 reductase domain (ferredoxin reductase-like sequence) containing a flavin mononucleotide (FMN) that is an coenzyme, and an iron-sulfur protein domain (ferredoxin-like sequence).

The fused cytochrome P450 monooxygenase contains a peptide which has a function similar to that of a reductase domain contained in the P450RhF and which is linked to the C-terminus of the P450 protein with a linker portion disposed therebetween.

Examples of the peptide include .(a) a peptide containing the amino acid sequence set forth in SEQ ID NO: 70; (b) a peptide containing an amino acid sequence prepared by adding one or more amino acid residues to the amino acid sequence set forth in SEQ ID NO: 70, removing one or more amino acid residues from this amino acid sequence, or replacing one or more amino acid residues of this amino acid sequence with other residues; and (c) a peptide encoded by a DNA that is hybridized with a DNA containing the amino acid sequence set forth in SEQ ID NO: 69 or a DNA complementary to this DNA under stringent conditions.

The peptide specified in Item (a) is one containing a domain similar to the reductase domain (a region containing the NADH-P450 reductase domain and the iron-sulfur protein domain) contained in the P450RhF.

The peptide specified in Item (b) is one that is prepared by introducing a small number of amino acid mutations into the peptide specified in Item (a) such that functions of the peptide specified in Item (a) are not lost. Examples of the amino acid mutations include natural amino acid mutations and artificial amino acid mutations. Examples of a technique for introducing such artificial amino acid mutations include a procedure for the construction of site-specific mutations (Nucleic acids res. 10, 6487-6500, 1982) and the technique is not limited to the procedure. The number of the amino acid mutations is not particularly limited and any number of the amino acid mutations may be used as long as the functions of the peptide specified in Item (a) are not lost. The number of the amino acid mutations is preferably 20 or less and more preferably ten or less.

The peptide specified in Item (c) is one, prepared by the hybridization between DNAs, having the same function as that of the peptide specified in Item (a). The term "stringent conditions" specified in Item (c) means conditions under which only specific hybridization occurs but no nonspecific hybridization occurs. The conditions are usually approximately "1 × SSC, 0.1% SDS, and 37°C", preferably approximately "0.5 × SSC, 0.1% SDS, and 42°C", and more preferably approximately "0.2 × SSC, 0.1% SDS, and 65°C". A DNA prepared by hybridization usually has high homology with DNAs used for the hybridization. The term "high homology" means a homology of 60% or more, preferably a homology of 75% or more, and more preferably a homology of 90% or more.

The linker portion is used to fuse the two proteins (peptides) and any linker portion that does not remove functions of the proteins may be used. Preferable examples of the linker portion include (d) a peptide containing the amino acid sequence set forth in SEQ ID NO: 68; (e) a peptide containing an amino acid sequence prepared by adding one or more amino acid residues to the amino acid sequence set forth in SEQ ID NO: 68, removing one or more amino acid residues from this amino acid sequence, or replacing one or more amino acid residues of this amino acid sequence with other residues; and (f) a peptide encoded by a DNA that is hybridized with a DNA containing the nucleotide sequence set forth in SEQ ID NO: 67 or a DNA complementary to this DNA under stringent conditions.

The peptide specified in Item (d) is one present between the P450 protein and reductase domain of P450RhF.

The peptide specified in Item (e) is one that is prepared by introducing a small number of amino acid mutations into the peptide specified in Item (d) such that functions of the linker portion are not lost. Examples of the amino acid mutations include natural amino acid mutations and artificial amino acid mutations. Examples of a technique for introducing such artificial amino acid mutations include a procedure for the construction of site-specific mutations (Nucleic acids res. 10, 6487-6500, 1982) and the technique is not limited to the procedure. The number of the amino acid mutations is not particularly limited and any number of the amino acid mutations may be used as long as the functions of the linker portion are not lost. The number of the amino acid mutations is preferably five or less, more preferably three or less, most preferably one.

The peptide specified in Item (f) is one, prepared by the hybridization between DNAs, having the same function as that of the peptide specified in Item (d). The term "stringent conditions" specified in Item (f) has the same meaning as that of the term "stringent conditions" specified in Item (c).

The P450 protein is not particularly limited. Examples of the P450 protein include a protein produced using a DNA isolated from DNAs, extracted from a bacterial genomic DNA library or an environmental sample, by an ordinary method such as a PCR method and a protein produced using a hybrid gene or a gene prepared by introducing mutations into a known P450 gene by a PCR random mutation method or a DNA shuffling method. The P450 protein may originate from a bacterium, a plant, or an animal and preferably originates from such a bacterium in particular. A protein originating from Acinetobacter calcoaceticus is a preferable example of the P450 protein. Other examples of the P450 protein include the proteins specified in Section "6. Novel P450".

### 9. Production of oxidized compound using fused P450 monooxygenase

The P450 protein specified in Section "8. Novel Fused Cytochrome P450 Monooxygenase" serves as a catalyst and thus an oxidized compound can be produced using the P450 protein. Examples of a method for producing such an oxidized compound include a method for producing the oxidized compound using a host cell into which an expression vector containing a gene encoding the fused P450 monooxygenase has been introduced and a method for producing the oxidized compound using the purified fused P450 monooxygenase.

Both methods do not require the presence of electron transfer proteins such as ferredoxins and ferredoxin reductases (reductases) because the fused P450 monooxygenase contains the electron transfer protein. Therefore, if the oxidized compound is produced using the host cell, a gene encoding the electron transfer protein need not be coexpressed. If the oxidized compound is produced using the purified fused P450 monooxygenase, an additional electron transfer protein need not be used. The fused P450 monooxygenase can be purified by a method similar to the method specified in Section "7. Production of oxidized compound using P450".

Any compound catalyzed by the fused P450 monooxygenase can be used as a substrate in the present production method. Examples of an organic compound used as such a substrate include alkanes (linear hydrocarbons), alkenes (linear hydrocarbons containing unsaturated bonds), cyclic hydrocarbons, alkyl aromatics, and alkenyl aromatics. According to the present production method, an oxidized compound can be produced by the hydroxylation of a terminal methyl group or the epoxidation of a terminal olefin group. In particular, 1-hexanol, 1-heptanol, 1-octanol, 1-decanol, 1,2-epoxyoctane, cyclohexanol, 4-phenyl-1-butanol, 2-phenethyloxirane, or 4-benzofran-2-yl-butane-1-ol can be produced from n-hexane, n-heptane, n-octane, n-decane, 1-octene, cyclohexane, n-butylbenzene, 4-phenyl-1-butene, or 2-n-butylbenzofran, respectively.

### Examples

The present invention will now be described in detail with reference to examples. The present invention is not limited the examples.

### [Example 1] Preparation of environmental DNAs

In order to obtain P450 genes, various DNAs were extracted from environmental samples described below.

### 1. Samples obtained from the waters off Palau or Ponape

The following technique has been disclosed: a technique for immersed a carrier containing a culture medium in seawater and then efficiently recovering DNAs from microorganisms attached to the carrier (Japanese Unexamined Patent Application Publication No. 2003-334064). In this example, sterile artificial sponges were immersed in an NSW culture medium (0.1% NH₄NO₃, 0.002% ferric citrate, 0.002% K₂HPO₄, 0.05% yeast extract solution, 80% filtered seawater, and 1.5% agar), whereby the medium was allowed to permeate the artificial sponges. The agar in the medium was then solidified. The artificial sponges were placed in positions 5 to 6 m above the bottom of the waters off State of Ponape, Federated States of Micronesia, or the bottom of the waters off Republic of Palau. The artificial sponges were recovered three days later after the placement. The artificial sponges having microorganisms attached thereto were frozen and then crushed. DNAs were extracted from the microorganisms attached to the crushed sponges using Dneasy Plant Kit (Qiagen).

### 2. Freshwater samples

A sample of groundwater was obtained from an oil storage nucleotide in Kuji-shi, Iwate-ken, and a sample of spring water was obtained from Toyotomi Spring in Teshio-gun, Hokkaido. Microorganisms present in those samples were selectively concentrated using filters (GV type, 0.22 µm, 47 mm, Millipore). To each of the filters, 5 ml of a 10 mM Tris-HCl buffer solution (pH 8.0, 1mM EDTA, 0.35 M sucrose) and 200 µl of a 10 mg/ml proteinase K solution (a 10 mM Tris-HCl buffer solution) were added. The microorganisms were then incubated at 37°C for 30 minutes. After the incubation was finished, the solution mixture was vigorously stirred, whereby the microorganisms were suspended in the solution mixture. After precipitates were removed from the solution mixture, 7.5 ml of a lysing solution (a 100 mM Tris-HCl buffer solution, pH 8.0, 0.3 M NaCl, 20 mM EDTA, 2% sodium dodecyl sulfate) was added to the resulting solution mixture. The obtained mixture was vigorously stirred. To the stirred mixture, 10 ml of a solution in which the ratio of phenol to chloroform to isoamyl alcohol is 50 : 49 : 1 was added. After five minutes passed, this mixture was subjected to centrifugal separation (5000 rpm and 15 min) and the upper phase was then recovered. A twofold volume of 99% ethanol was added to the upper phase and this mixture was stirred and then cooled at -80°C for two hours. This mixture was subjected to centrifugal separation (15000 rpm and 15 min), whereby DNAs were precipitated. Cool 70% ethanol was added to this mixture, which was further subjected to centrifugal separation (15000 rpm and 15 min), whereby a precipitate fraction containing the DNAs was obtained.

### 3. Soil sample

A sample of oil-contaminated soil was obtained from an oil well in Nishiyama-cho, Niigata-ken. The following solutions were added to 5 g of the soil sample: 5 ml of a 10 mM Tris-HCl buffer solution (pH 8.0, 1mM EDTA, 0.35 M sucrose) and 200 µl of a 10 mg/ml proteinase K solution (a 10 mM Tris-HCl buffer solution). The mixture was subjected to incubation at 37°C for 30 minutes. After the incubation was finished, the mixture was vigorously stirred, whereby microorganisms were suspended in the mixture. After precipitates were removed from the mixture, 7.5 ml of a lysing solution (a 100 mM Tris-HCl buffer solution, pH 8.0, 0.3 M NaCl, 20 mM EDTA, 2% sodium dodecyl sulfate) was added to the resulting mixture. The obtained mixture was vigorously stirred. To the stirred mixture, 10 ml of a solution in which the ratio of phenol to chloroform to isoamyl alcohol is 50 : 49 : 1 was added. After five minutes passed, this mixture was subjected to centrifugal separation (5000 rpm and 15 min) and the upper phase was then recovered. A twofold volume of 99% ethanol was added to the upper phase and this mixture was stirred and then cooled at -80°C for two hours. This mixture was subjected to centrifugal separation (15000 rpm and 15 min), whereby DNAs were precipitated. Cool 70% ethanol was added to this mixture, which was further subjected to centrifugal separation (15000 rpm and 15 min), whereby a precipitate fraction containing the DNAs was obtained.

### [Example 2] Design of PCR primers

The inventors have discovered that there are two conserved regions, that is, MFIAMDPP (located close to the N-terminus) and HRCMGNRL (located close to the C-terminus) in an alignment of three amino acid sequences, that is, CYP153A1 (accession no. AJ311718), CYP153A2 (accession no. AE005680), and CYP153A13a (SEQ ID NO: 58), belonging to the CYP153A subfamily of the disclosed P450 superfamily. The inventors designed four types of primers on the basis of amino acid sequences (MFIAMDPP and HRCMGNRL) in the two conserved regions. The designed primers were 5'-ATGTTYATHGCNATGGAYCCNC-3' (SEQ ID NO: 53) located close to the conserved amino acid sequence MFIAMDPP (located close to the N-terminus) (SEQ ID NO: 51), 5'-CNGGRTCCATNGCDATRAACAT-3' (SEQ ID NO: 54) that is a complementary chain thereof, 5'-NARNCKRTTNCCCATRCANCKRTG-3' (SEQ ID NO: 55) located close to the conserved amino acid sequence HRCMGNRL (located close to the C-terminus) (SEQ ID NO: 52), and 5'-CAYMGNTGYATGGGNAAYMGNYT-3' (SEQ ID NO: 56) that is a complementary chain thereof. In these primer sequences, Y is C or T; H is A, C, or T; N is A, T, G, or C; R is A or G; D is A, G, or T; K is G or T; and M is A or C.

### [Example 3] Amplification of P450 gene

PCR amplification reactions were performed using the primer designed in Example 2 in such a manner that the DNAs prepared in Example 1 were used as templates. The primer of SEQ ID NO: 53 was used in combination with and the primer of SEQ ID NO: 55. Each reaction solution was prepared as follows: 0.5 unit of DNA polymerase Amplitaq Gold (Applied Biosystems), 5 µl of an attached polymerase buffer solution, 5 µl of a 2 mM dNTP solution, 4 µl of a 50 pmol/µl solution of one of the primers, 4 µl of a 50 pmol/µl solution of the other one, and 100 ng of one of the DNAs obtained from the environmental samples were mixed together and sterile water was then added to the mixture such that 50 µl of the reaction solution was obtained. In each reaction, denaturation was performed at 94°C for 10 minutes, a cycle of treatment was repeated 35 times, and additional treatment was then finally performed at 72°C for ten minutes, the treatment cycle being performed at 94°C for 30 seconds, 58°C for 45 seconds, and then 72°C for one minute. The amplification was confirmed by electrophoresis using a 1.5% agarose gel. The amplified DNAs with a predicted length (about 0.85 Kbp) were recovered from the agarose and then purified with a QIAquick Gel Extraction kit (Qiagen).

### [Example 4] Isolation of P450 gene from Alcanivorax borkumensis SK2

The inventors isolated DNA fragments containing CYP153A13a (SEQ ID NO: 58) from a strain of Alcanivorax borkumensis SK2 (DSM 11573) that was a marine bacterium utilizing an alkane by the procedure below.

Two primer sequences, SEQ ID NO: 59 (5'-GCCGATGGAGTTTATGGGCAAT-3') AND SEQ ID NO: 60 (5'-GTACCACATCACCACCTTGTCGCC-3'), were designed on the basis of the sequence CYP153A1 (accession no. AJ311718) contained in a P450 monooxygenase present in a strain of Acinetobacter calcoaceticus EB104 that is a bacterium utilizing a long-chain n-alkane. A PCR amplification reaction was then performed using a genomic DNA extracted from the Alcanivorax borkumensis SK2 strain as a template. In the reaction, 16 types of buffer solutions included in a PCR Optimizer kit (Invitrogen) were used and a cycle of treatment was repeated 40 times, the treatment cycle being performed at 94°C for one minute, 55°C for two minutes, and then 72°C for three minutes. The amplification was confirmed by electrophoresis using a 2% agarose gel. This resulted in the confirmation of amplified P450 gene fragments (about 250 bp). A P450 monooxygenase gene was cloned by hybridization using the amplified P450 gene fragments as probes. The Alcanivorax borkumensis-derived genomic DNA was partially cleaved into fragments with the restriction enzyme Sau3AI and the fragments were linked to the BamHi sites of the cosmid vector SuperCos 1 and then packaged into phages. Strains of Escherichia coli XL1-Blue MR were infected with the phages and colonies resistant to antibody Ampicillin were picked up, whereby a cosmid library was prepared. The colonies were hybridized using AlkPhos Direct Labelling and Detection System (Amersham Biosciences Corp.) according to a protocol attached thereto. Labeled positive clones were recovered and then subjected to Southern hybridization using AlkPhos Direct Labelling and Detection System (Amersham Biosciences Corp.) according to a protocol attached thereto, resulting in the detection of bands hybridized with the P450 monooxygenase gene fragments. The analysis of the DNA sequence of one of the obtained clones showed the presence of a full length of a cloned P450 monooxygenase gene (CYP153A13a containing the DNA sequence set forth in SEQ ID NO: 58 and the amino acid sequence set forth in SEQ ID NO: 57) belonging to the CYP153 family.

### [Example 5] Preparation of hybrid gene

The following fragments were amplified from a CYP153A13a gene (SEQ ID NO: 58) cloned from a strain of Alcanivorax borkumensis SK2 by a PCR: a DNA fragment (hereinafter referred to as "5-arm") ranging from an initiation codon to a portion encoding a conserved region (the amino acid sequence MFIAMDPP) and a DNA fragment (hereinafter referred to as "3-arm") ranging from a termination codon to a portion encoding a second conserved region (the amino acid sequence HRCMGNRL). The 5-arm was PCR-amplified as described below. A reaction solution used was prepared as follows: 0.5 unit of DNA polymerase Amplitaq Gold (Applied Biosystems), 5 µl of an attached polymerase buffer solution, 5 µl of a 2 mM dNTP solution, 4 µl of a 50 pmol/µl solution of the primer of SEQ ID NO: 61 (5'-CACCATGTCAACAGTTCAAGTA-3'), 4 µl of a 50 pmol/µl solution of the primer of SEQ ID NO: 54, and 100 ng of a cosmid vector containing a cloned CYP153A13a gene were mixed together and sterile water was then added to the mixture such that 50 µl of the reaction solution was obtained. In the reaction, denaturation was performed at 94°C for 10 minutes, a cycle of treatment was repeated 30 times, and additional treatment was then finally performed at 72°C for ten minutes, the treatment cycle being performed at 94°C for 30 seconds, 58°C for 45 seconds, and then 72°C for one minute. The amplified 5-arm fragments were confirmed by electrophoresis using a 1.5% agarose gel. For the amplification of the 3-arm fragment, the primer of SEQ ID NO: 62 (5'-TGATTATTTTTTAGCCGTCAACT-3') and the primer of SEQ ID NO: 56 were used instead of the primer of SEQ ID NO: 61 and the primer of SEQ ID NO: 54. The amplification was confirmed by electrophoresis using a 1.5% agarose gel. DNAs with a predicted length were recovered from the agarose and then purified with a QIAquick Gel Extraction kit (Qiagen). Each hybrid gene was prepared, by PCR, from the 5- and 3-arm fragments and a PCR product based on the DNA prepared from one of the environmental samples in Example 3. A reaction solution used was prepared as follows: 0.5 unit of DNA polymerase Amplitaq Gold (Applied Biosystems), 5 µl of an attached polymerase buffer solution, 5 µl of a 2 mM dNTP solution, 4 µl of a 50 pmol/µl solution of the primer of SEQ ID NO: 61, 4 µl of a 50 pmol/µl solution of the primer of SEQ ID NO: 62, 20 ng of the PCR product based on the DNA prepared from the environmental sample in Example 3, 5 mg of the 5-arm fragment, and 5 mg of the 3-arm fragment were mixed together and sterile water was then added to the mixture such that 50 µl of the reaction solution was obtained. In the reaction, denaturation was performed at 94°C for 10 minutes, a cycle of treatment was repeated 30 times, and additional treatment was then finally performed at 72°C for ten minutes, the treatment cycle being performed at 94°C for 30 seconds, 58°C for 45 seconds, and then 72°C for one minute. The amplification was confirmed by electrophoresis using a 1.5% agarose gel. DNAs with a predicted length (about 1.4 Kbp) were recovered from the agarose and then purified with a QIAquick Gel Extraction kit (Qiagen). Purified hybrid genes were cloned into sequence vectors (pENTR/SD/D-TOPO) (Invitrogen) using GATEWAY System pENTR Directional TOPO Cloning Kits (Invitrogen).

### [Example 6] Analysis of DNA sequence

The plasmid vectors containing the hybrid genes prepared in Example 5 were transformed into strains of Escherichia coli (One Shot TOP10 Chemically Component E. coli available from Invitrogen). Colonies of the Escherichia coli strains were randomly selected and then inoculated into LB liquid culture media (1% tryptone, 0.5% yeast extract, and 0.5% NaCl). Plasmids were extracted therefrom with a QIAprep Spin Miniprep kit (Qiagen). Sequence reactions were performed by the dideoxy chain termination method using a Big Dye Terminator Cycle Sequencing FS Ready Reaction kit (Applied Science). The nucleotide sequences of reaction products were determined with ABI prism 3730 DNA Analyzer (Applied Science). Gene sequences having the common sequence FXXGXR/HXCXG, characteristic of P450, 50 to 60 residues apart from the C-terminuses of predicted amino acid sequences were selected from obtained gene sequences. The hybrid genes had the amino acid sequences set forth in SEQ ID NOS: 1 to 25 and the nucleotide sequences set forth in SEQ ID NOS: 26 to 50 in addition to the common sequence.

### [Example 7] Analysis of sequences

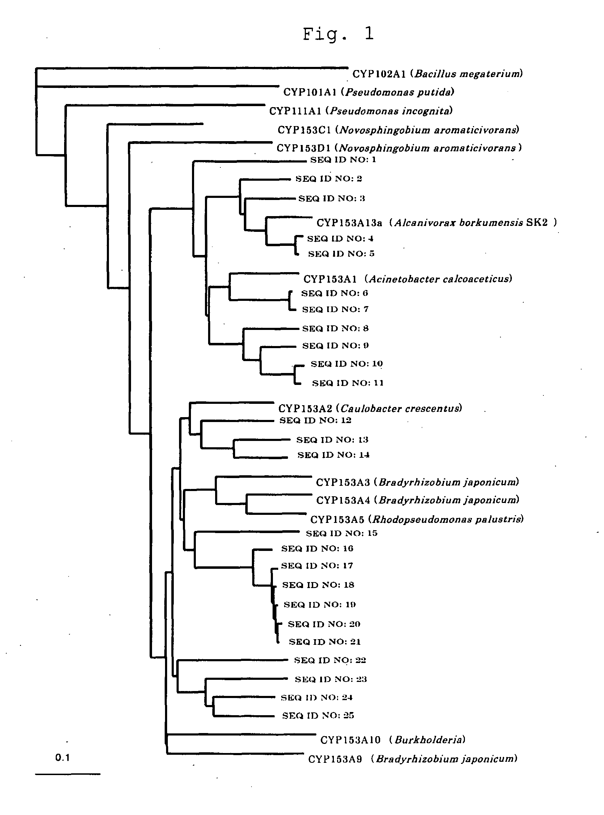

The amino acid sequences (SEQ ID NOS: 1 to 25) of the hybrid P450 genes obtained in Example 6 were compared with amino acid sequences between conserved regions (SEQ ID NOS: 51 and 52) in known P450 amino acid sequences. The comparison between these amino acid sequences were performed using an alignment software, Clustal W (Higgins, D. G., Thompson, J. D., and Gibson, T. J., Methods Enzymol. 266, 383-402, 1996). The known P450 sequence was obtained by NCBI Blast searching (http://ncbi.nlm.nih.gov/). Fig. 1 shows the compared sequences in a genealogical tree. Table 1 summarizes the homology between the sequences and four types of known CYP153A subfamilies. As is clear from Fig. 1 and Table 1, the amino acid sequences between the conserved regions in the amino acid sequences of SEQ ID NOS: 1 to 25 are different from the sequences between the conserved regions in the known P450 amino acid sequences and thus belong to new classes. Furthermore, as is clear from Table 1, novel P450 genes can be obtained by PCR using DNAs, originating from the microorganisms extracted from the various environmental samples obtained from Ponape seawater, Palau seawater, Kuji underwater, Hokkaido spring water, or oil-contaminated soil in Niigata, as templates.

**[Table 1]**

| Homology (%) | | | | | |
|---|---|---|---|---|---|
| SEC1 ID NOS | CYP153A13a | CYP153A1 | CYP153A2 | CYP153A3 | Sources of Template DNAs |
| 1 | 65 | 67 | 55 | 49 | Niigata Soil |
| 2 | 79 | 71 | 57 | 51 | Niigata Soil |
| 3 | 79 | 70 | 57 | 51 | Niigata soil |
| 4 | 87 | 73 | 56 | 49 | Ponape Seawater |
| 5 | 88 | 74 | 57 | 50 | Ponape Seawater |
| 6 | 72 | 79 | 59 | 52 | Ponape Seawater |
| 7 | 72 | 79 | 57 | 51 | Ponape Seawater |
| 8 | 72 | 70 | 57 | 49 | Palau Seawater |
| 9 | 73 | 75 | 58 | 51 | Niigata Soil |
| 10 | 71 | 73 | 56 | 51 | Niigata Soil |
| 11 | 71 | 73 | 56 | 50 | Niigata Soil |
| 12 | 53 | 56 | 75 | 66 | Niigata Soil |
| 13 | 55 | 54 | 69 | 66 | Kuji Underwater |
| 14 | 52 | 55 | 71 | 64 | Niigata Soil |
| 15 | 52 | 52 | 66 | 64 | Palau Seawater |
| 16 | 54 | 57 | 72 | 65 | Kuji Underwater |
| 17 | 56 | 57 | 69 | 63 | Kuji Underwater |
| 18 | 55 | 57 | 70 | 64 | Kuji Underwater |
| 19 | 55 | 57 | 69 | 63 | Palau Seawater |
| 20 | 54 | 57 | 70 | 63 | Kuji underwater |
| 21 | 55 | 57 | 70 | 63 | Kuji Underwater |
| 22 | 57 | 58 | 67 | 57 | Hokkaido Spring Water |
| 23 | 52 | 55 | 68 | 59 | Niigata Soil |
| 24 | 53 | 56 | 70 | 62 | Niigata soil |
| 25 | 54 | 57 | 71 | 63 | Kuji Underwater |

### [Example 8] Expression of hybrid genes

Three of the hybrid genes containing the novel sequences shown in Example 7 were expressed in strains of Escherichia coli and functions thereof were then investigated. Three types of genes (SEQ ID NOS: 4, 5, and 9) encoding P450 were inserted into protein-expressing plasmid vectors (pDEST14 produced by Invitrogen) by the LR Clonase reaction using a Gateway system. The resulting vectors were transformed into strains of Escherichia coli DH5α (Invitrogen). The strains were inoculated into LB liquid culture media. Plasmids were extracted from the strains using QIAprep Spin Miniprep Kits (Qiagen). The plasmids obtained were transformed into strains of Escherichia coli BL21-Ai (Invitrogen). These strains were inoculated into 5 ml of LB liquid culture media supplemented with a solution of Ampicillin (Wako Pure Chemical Industries) with a final concentration of 50 µg/ml and then cultured overnight. Subsequently, 1 ml of culture solutions obtained from these media were added to 100 ml of 100 LB liquid culture media supplemented with an Ampicillin solution with a final concentration of 50 µg/ml and these strains were further cultured at 37°C. At the beginning of logarithmic growth, a 1 mM solution of 5-aminolevulin acid hydrochloride (Wako Pure Chemical Industries) and a 0.5 mM FeCl₃ solution were added to these media. After these strains were cultured at 25°C for 30 minutes, a 0.2% solution of arabinose (Wako Pure Chemical Industries) was added to these media and these strains were further cultured. After the culture (at 25°C for 16 hours) was finished, these strains were collected with a centrifugal separator, suspended in PBS (phosphate-buffered saline; 137 mM NaCl, 10 mM Na₂HPO₄, and 2.68 mM KCl) containing 20% glycerol (Wako Pure Chemical Industries), disrupted with an ultrasonic disruptor, and then subjected to centrifugal separation, whereby supernatant liquids were obtained. One of the supernatant liquids that was obtained from the Escherichia coli strains containing the plasmid vector containing the gene encoding SEQ ID NO: 4 was named Crude Extract Solution A, another one obtained from the Escherichia coli strains containing the plasmid vector containing the gene encoding SEQ ID NO: 5 was named Crude Extract Solution B, and the other one obtained from the Escherichia coli strains containing the plasmid vector containing the gene encoding SEQ ID NO: 9 was named Crude Extract Solution C.

### [Example 9] Analysis of functions by CO difference spectrometry

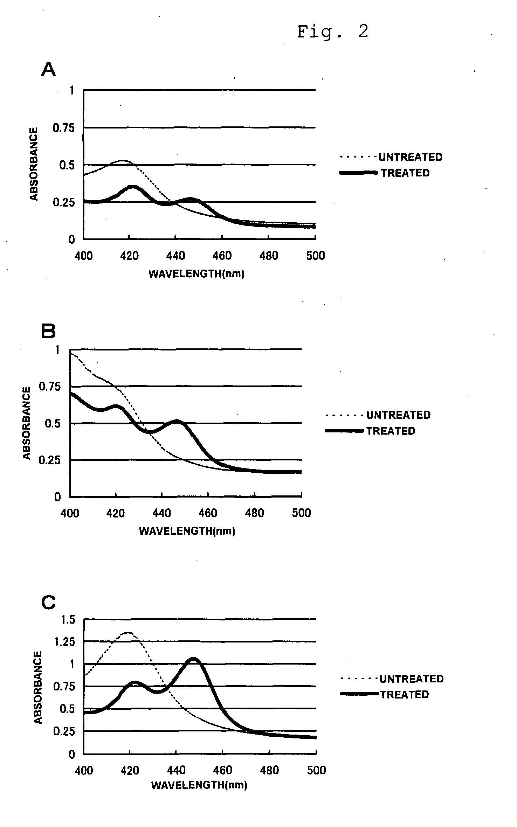

It is known that P450 has a CO difference spectrum with a maximum at 450 nm after P450 is treated with sodium dithinaite (Na₂S₂O₄) and then carbon monoxide. Thus, carbon monoxide was applied to 1 ml of Crude Extract Solutions A to C prepared in Example 8 for one minute such that several bubbles per second are created, 2 mg of sodium dithinaite was mixed with each of Crude Extract Solutions A to C, the mixtures were allowed to stand for five minutes, and the absorption spectra of the mixtures were then measured, whereby untreated or treated Crude Extract Solutions A to C were subjected to spectrum analysis. As shown in Fig. 2, the 450 nm absorbance of Crude Extract Solutions A to C treated with carbon monoxide under reductive conditions is greater than that of untreated Crude Extract Solutions A to C. This confirms the presence of soluble, functional P450 in Crude Extract Solutions A to C. This shows that the three amino acid sequences (SEQ ID NOS: 4, 5, and 9) are proteins serving as P450.

### [Example 10] Isolation of putidaredoxin and putidaredoxin reductase originating from Pseudomonas putida

In order to catalyze oxidation, the novel P450, as well as ordinary microbial P450, prepared as described above probably requires a ferredoxin reductase and ferredoxin that are electron transfer proteins. The inventors have developed a technique for using a putidaredoxin and putidaredoxin reductase originating from Pseudomonas putida as electron transfer proteins and cloned genes encoding such electron transfer proteins.

For the putidaredoxin, primers (the sequence 5'-CGTCTCCCATGTCTAAAGTAGTGTATGTGT-3' set forth in SEQ ID NO: 63 and the sequence 5'-CGTCTCATCGATTACCATTGCCTATCGGGA-3' set forth in SEQ ID NO: 64) containing restriction enzyme sites were designed from the gene (accession no. D00528) encoding the putidaredoxin and then cloned by PCR using a genomic DNA originating from Pseudomonas putida as a template. A reaction solution used was prepared as follows: 0.5 unit of DNA polymerase Amplitaq Gold (Applied Biosystems), 5 µl of an attached polymerase buffer solution, 5 µl of a 2 mM dNTP solution, 4 µl of a 50 pmol/µl solution of one of the primers, 4 µl of a 50 pmol/µl solution of the other one, and 100 ng of the prepared DNA were mixed together and sterile water was then added to the mixture such that 50 µl of the reaction solution was obtained. In the reaction, denaturation was performed at 94°C for 10 minutes, a cycle of treatment was repeated 30 times, and additional treatment was then finally performed at 72°C for ten minutes, the treatment cycle being performed at 94°C for 30 seconds, 58°C for 45 seconds, and then 72°C for one minute. The amplification was confirmed by electrophoresis using a 1.5% agarose gel. The amplified DNAs with a predicted length were recovered from the agarose and then purified with a QIAquick Gel Extraction kit (Qiagen).

For the putidaredoxin reductase, primers (the sequence 5'-CGTCTCCCATGAACGCAAACGACAACGTGG-3' set forth in SEQ ID NO: 65 and the sequence 5'-CGTCTCATCGATCAGGCACTACTCAGTTCA-3' set forth in SEQ ID NO: 66) containing restriction enzyme sites were designed from the gene (accession no. D00528) encoding the putidaredoxin reductase and then cloned by PCR using a genomic DNA originating from Pseudomonas putida as a template. A reaction solution used was prepared as follows: 0.5 unit of DNA polymerase Amplitaq Gold (Applied Biosystems), 5 µl of an attached polymerase buffer solution, 5 µl of a 2 mM dNTP solution, 4 µl of a 50 pmol/µl solution of one of the primers, 4 µl of a 50 pmol/µl solution of the other one, and 100 ng of the prepared DNA were mixed together and sterile water was then added to the mixture such that 50 µl of the reaction solution was obtained. In the reaction, denaturation was performed at 94°C for 10 minutes, a cycle of treatment was repeated 30 times, and additional treatment was then finally performed at 72°C for ten minutes, the treatment cycle being performed at 94°C for 30 seconds, 58°C for 45 seconds, and then 72°C for one minute. The amplification was confirmed by electrophoresis using a 1.5% agarose gel. The amplified DNAs with a predicted length were recovered from the agarose and then purified with a QIAquick Gel Extraction kit (Qiagen).

The full-length genes, isolated by PCR, encoding the putidaredoxin or the putidaredoxin reductase were treated with the restriction enzyme BsmBI. The expression vectors pET-28a (Novagen) were treated with the restriction enzymes Sal I and Nco I. The genes and vectors were ligated (Ligation High produced by Toyobo) and then transformed into strains of Escherichia coli JM109 (Takara Shuzo). The strains were inoculated into an LB liquid culture medium and plasmids were extracted therefrom with a QIAprep Spin Miniprep kit (Qiagen).

### [Example 11] Activity to oxygenate substrate

The three amino acid sequences (SEQ ID NOS: 4, 5, and 9), which were the novel P450 expressing the soluble, functional proteins as described in Example 9, were investigated if the amino acid sequences have the activity to oxygenate a substrate.

The expression vectors containing the putidaredoxin, originating from Pseudomonas putida, isolated in Example 10 were transformed into strains of Escherichia coli Rosseta DE3 (Novagen) and the expression vectors containing the putidaredoxin reductase originating from Pseudomonas putida were transformed into strains of Escherichia coli BL21 DE3 pLysS (Novagen). These strains were inoculated into 5 ml of LB liquid culture media and then cultured overnight. The following liquid was added to 100 ml of a TB liquid culture medium (1.2% tryptone, 2.4% yeast extract, 0.4% glycerol, 0.231% KH₂PO₄, and 1.254% K₂HPO₄) supplemented with a kanamycin solution with a final concentration of 25 µg/ml: 1 ml of a culture liquid obtained from the culture medium inoculated with the strains transformed from the expression vectors containing the putidaredoxin. The following liquid was added to 100 ml of a TB liquid culture medium supplemented with a chloramphenicol solution with a final concentration of 50 µg/ml and a kanamycin solution with a final concentration of 25 µg/ml: 1 ml of a culture liquid obtained from the culture medium inoculated with the strains transformed from the expression vectors containing the putidaredoxin reductase. The strains in the culture liquids were cultured at 37°C. At the beginning of logarithmic growth, an isopropyl-β-D-thiogalactopyranoside (IPTG) solution was added to these media such that the these media had a final IPTG concentration of 0.5 mM. The strains were further cultured. After the culture (at 30°C for 16 hours) was finished, these strains were collected with a centrifugal separator, suspended in PBS containing 20% glycerol, disrupted with an ultrasonic disruptor, and then subjected to centrifugal separation, whereby supernatant liquids, that is, crude extract solutions, were obtained. These crude extract solutions were mixed with crude extract solutions each containing one of the three types of novel P450 (SEQ ID NOS: 4, 5, and 9) in nearly equal proportions, whereby crude enzyme solutions were obtained. The crude enzyme solution consisting of the crude extract solution containing the novel P450 of SEQ ID NO: 4 and the crude extract solutions containing the two electron transfer proteins was named Crude Enzyme Solution A, the crude enzyme solution consisting of the crude extract solution containing the novel P450 of SEQ ID NO: 5 and the crude extract solutions containing the two electron transfer proteins was named Crude Enzyme Solution B, the crude enzyme solution consisting of the crude extract solution containing the novel P450 of SEQ ID NO: 9 and the crude extract solutions containing the two electron transfer proteins was named Crude Enzyme Solution C, and the crude enzyme solution consisting of the crude extract solutions containing the two electron transfer proteins was named Crude Enzyme Solution D. Crude Enzyme Solutions A to D were used for an enzyme reaction. Before the enzyme reaction was started, a substrate was added to each crude enzyme solution such that the crude enzyme solution had a final substrate concentration of 1 mM, the mixture was allowed to stand for 10 minutes at 25°C, NADP was then added to the mixture such that the mixture had a final NADP concentration of 5 mM. The substrate was octane, decane, or 1-decene. The enzyme reaction was performed at 25°C for 12 hours and then terminated in such a manner that hydrochloric acid was added to the reaction mixture such that the reaction mixture had a final hydrochloric acid concentration of 0.2 N. Ethyl acetate was added to the resulting crude enzyme solution and a fraction extracted from the crude enzyme solution with ethyl acetate was analyzed with a gas chromatography mass spectrometer (GCMS-QP5050A manufactured by Shimadzu Corporation), whereby the presence of an oxidized compound derived from the substrate was investigated.

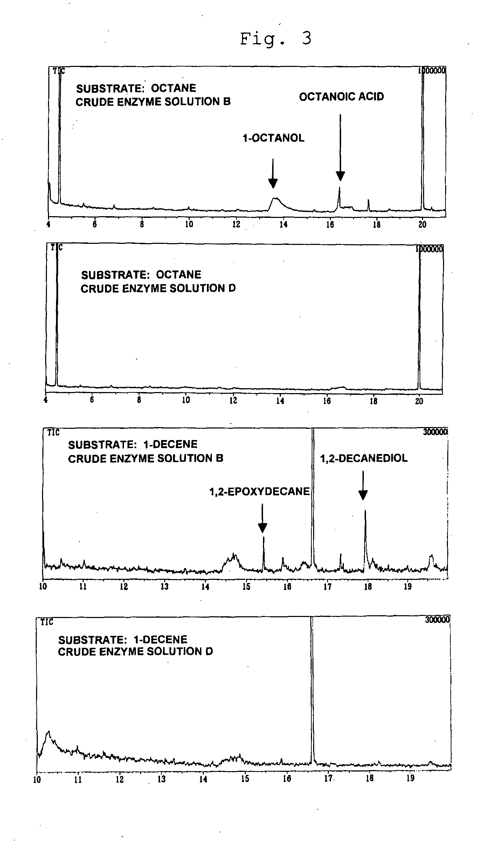

Fig. 3 shows the analysis of fractions extracted from reaction mixtures obtained by subjecting to octane or 1-decene to the enzyme reaction using Crude Enzyme Solution B or D. The fraction extracted from the reaction mixture obtained by the reaction of octane using Crude Enzyme Solution B contains 1-octanol and octanoic acid, which are oxidized compounds derived from octane. In contrast, the fraction extracted from that using Crude Enzyme Solution D, which contains no P450, contains no oxidized compound derived from octane. The fraction extracted from the reaction mixture obtained by the reaction of 1-decene using Crude Enzyme Solution B contains 1,2-epoxydecane and 1,2-decanediol, which are oxidized compounds derived from octane. In contrast, the fraction extracted from that obtained using Crude Enzyme Solution D, which contains no P450, contains no oxidized compound derived from 1-decene. These show that the novel P450 (SEQ ID NO: 5), obtained as described above, contained in Crude Enzyme Solution B has the activity to catalyze the oxidation of organic compounds such as octane and 1-decene.

Octane, decane, and 1-decene were subjected to the enzyme reaction using Crude Enzyme Solution A, B, C, or D and the reaction mixtures were analyzed. Table 2 shows detected oxidized compounds derived from the substrates.

**[Table 2]**

| Substrate | Crude Enzyme Solution A | Crude Enzyme Solution B | Crude Enzyme Solution C | Crude Enzyme Solution D |
|---|---|---|---|---|
| Octane | 1-octanol and octanoic acid | 1-octanol and octanoic acid | Octanoic acid | Not detected |
| Decane | Not detected | Decanoic acid | Not detected | Not detected |
| 1-decene | Not detected | 1,2-epoxydecane and 1,2-decanediol | Not detected | Not detected |

The oxidized compounds derived from octane were detected in the extract fractions obtained from Crude Enzyme Solutions A, B, and C. This shows that all the three types of novel P450 (SEQ ID NOS: 4, 5, and 9) contained in the crude enzyme solutions have oxygenation activity. The table shows that the novel P450 has the ability to catalyze the oxidation and/or epoxidation of alkanes, such as octane and decane, having different chain lengths.

### [Example 12] Preparation of plasmid for producing fusion protein having RhF reductase domain

In order to readily investigate the activity of the obtained hybrid P450 genes to oxidize various substrates by bioconversion using Escherichia coli hosts, an expression plasmid for producing a fusion protein containing P450 and an electron transfer protein domain (reductase domain) was prepared.

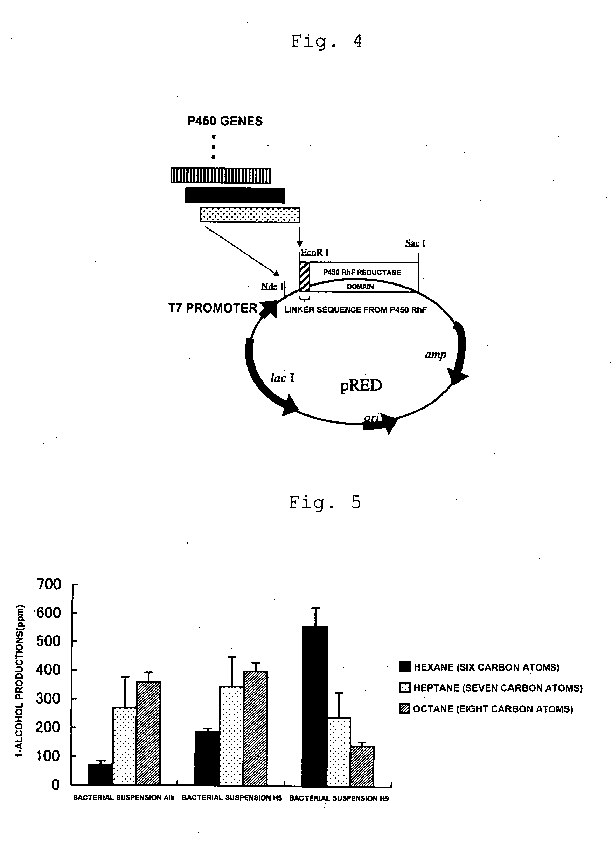

The following gene has been isolated from Rhodococcus sp. strain NCIMB 9784: a fused P450 monooxygenase (P450RhF) gene containing a single polypeptide chain containing a P450 protein domain (heme domain) and reductase domain fused with each other (Non-patent Document 8). A 1.0 kb DNA fragment containing the following gene was PCR-amplified using a primer (5'-GGGAATTCGTGCTGCACCGCCATCAACCG-3', SEQ ID NO: 71) containing a restriction enzyme domain originating from a template DNA and a primer (5'-GGGAGCTCTCAGAGGCGCAGGGCCAGGCG-3', SEQ ID NO: 72): a gene encoding a linker sequence (the nucleotide sequence set forth in SEQ ID NO: 67 and the amino acid sequence set forth in SEQ ID NO: 68) and reductase domain (the nucleotide sequence set forth in SEQ ID NO: 69 and the amino acid sequence set forth in SEQ ID NO: 70) contained in the P450RhF gene originating from Rhodococcus sp. strain NCIMB 9784. The amplified 1.0 kb DNA fragments were excised with EcoRI and SacI and then inserted into the EcoRI and SacI sites in the Escherichia coli vectors pET21a. (Novagen). The plasmids are hereinafter referred to as pRED. The map of pRED is shown in Fig. 4.

### [Example 13] Preparation of artificial fusion enzymes containing hybrid P450 genes and RhF reductase domains

In order to investigate the activity of the obtained hybrid P450 genes to oxidize various substrates by bioconversion using Escherichia coli hosts, artificial fusion enzymes containing the hybrid P450 genes were prepared by inserting the hybrid P450 genes into plasmid vectors pRED for producing fusion proteins. The fusion proteins have structures in which the hybrid P450 genes are linked to RhF reductase domains with linker portions.

Primers (5'-CACTAGTCATATGTCAACGATGTCAAGTACA-3' SEQ ID NO: 73 and 5'-CAGCACAATTGTTTTTTAGCCGTCAACTT-3' SEQ ID NO: 74) containing restriction enzyme sites were designed. Fragments of the P450 genes were PCR-amplified in such a manner that sequence vectors containing genes encoding the five types of novel P450 (SEQ ID NOS: 3, 5, 7, 9, and 11) prepared in Example 5 were used as templates. Termination codons were removed from the amplified P450 gene fragments. The obtained 1.4 kb DNA fragments were cleaved with restriction enzymes NdeI and MfeI and then inserted into the NdeI-EcoRI sites of a plasmid pRED. A plasmid vector containing the gene encoding SEQ ID NO: 3 was named pH3RED, a plasmid vector containing the gene encoding SEQ ID NO: 5 was named pH5RED, a plasmid vector containing the gene encoding SEQ ID NO: 7 was named pH7RED, a plasmid vector containing the gene encoding SEQ ID NO: 9 was named pH9RED, and a plasmid vector containing the gene encoding SEQ ID NO: 11 was named pH11RED. A plasmid vector pAlkRED containing a CYP153A13a cloned from a strain of Alcanivorax borkumensis SK2 was prepared.

### [Example 14] Experiments of converting alkanes (linear hydrocarbons) by use of fused P450 monooxygenases

The plasmid vectors pH3RED, pH5RED, pH7RED, pH9RED, pH11RED, and pAlkRED were transformed into strains of Escherichia coli BL21-AI. The strains were inoculated into 5 ml of LB liquid culture media with a final Ampicillin concentration of 50 µg/ml and then cultured overnight. To 100 ml of LB liquid culture media with a final Ampicillin concentration of 50 µg/ml, 1 ml of culture liquids obtained from those LB liquid culture media were added. The strains were cultured at 30°C. At the beginning of logarithmic growth, a 1 mM solution of 5-aminolevulin acid hydrochloride and a 0.5 mM FeCl₃ solution were added to these media. After these strains were cultured at 20°C for 30 minutes, a 0.2% arabinose solution was added to these media and these strains were further cultured. After the culture (at 20°C for 16 hours) was finished, these strains were collected with a centrifugal separator and then suspended in 10 ml of 100 mM phosphate buffers (pH 7.5). A bacterial suspension containing the pH3RED-expressed strains was named H3, a bacterial suspension containing the pH5RED-expressed strains was named H5, a bacterial suspension containing the pH7RED-expressed strains was named H7, a bacterial suspension containing the pH9RED-expressed strains was named H9, a bacterial suspension containing the pH11RED-expressed strains was named H11, and a bacterial suspension containing the pAlkRED-expressed strains was named HAlk.

To 1 ml of the bacterial suspensions, 0.25 ml of substrates were added and the mixtures were subjected to reactions. The substrates were n-hexane, n-heptane, n-octane, and n-decane. Hydrochloric acid was added to the mixtures such that the resulting mixtures had a final hydrochloric acid concentration of 0.2 N, whereby the reactions were terminated. To the mixtures, 0.25 ml of hexane was added. Hexane extract fractions were obtained from the mixtures and then analyzed with a gas chromatography mass spectrometer, whereby oxidized compounds derived from the substrates were identified and the amounts of the oxidized compounds were determined. Table 3 shows the amounts (contents) of the oxidized compounds produced by the oxidation using the bacterial suspensions.

**[Table 3]**

| (Substrates) Oxides | Bacterial Suspension Alk | Bacterial Suspension H3 | Bacterial Suspension H5 | Bacterial Suspension H7 | Bacterial Suspension H9 | Bacterial Suspension H11 |
|---|---|---|---|---|---|---|
| (n-Hexane) 1-Hexamol | 71±14 ppm | 148±8 ppm | 187±11 ppm | 232±26 ppm | 559±66 ppm | 48±2 ppm |
| (n-Heptane) 1-Heptanol | 269±109 ppm | 257±34 ppm | 345±106 ppm | 49±11 ppm | 239±88 ppm | 43±6 ppm |
| (n-Octanol) | 359±34 ppm | 301±39 ppm | 399±31 ppm | 163±14 ppm | 141±14 ppm | 129±6 ppm |
| (n-Decane) 1-Decanol | 75±53 ppm | 43±30 ppm | 28±31 ppm | 2.5±4.4 ppm | 42±14 ppm | 35±22 ppm |

The experiments of converting the linear hydrocarbons by bioconversion, as well as the experiments performed using the crude enzyme solutions prepared from the bacterial solutions (Example 11), show that the hybrid P450 monooxygenases catalyze the alcoholization of the n-alkanes such as hexane, octane, and decane.

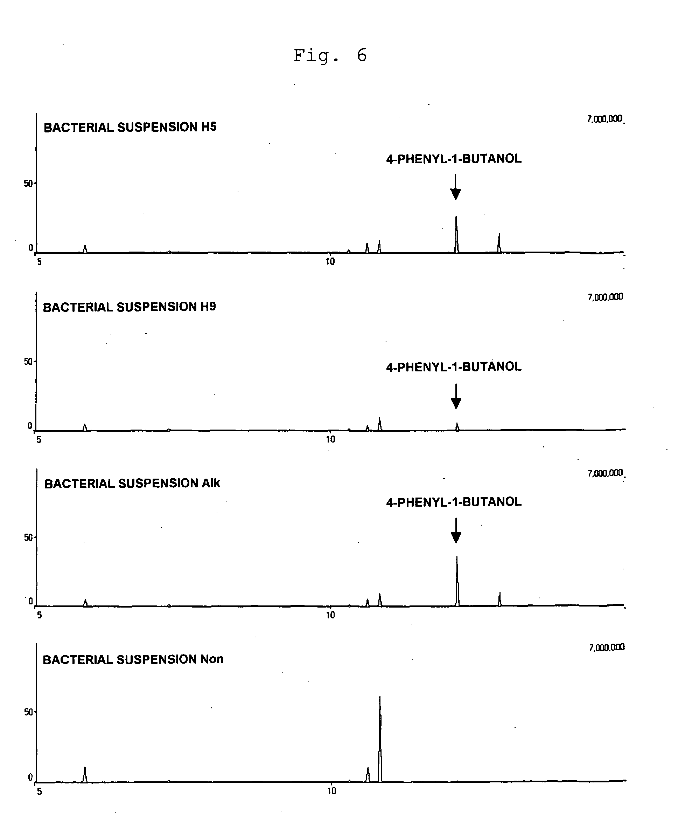

Fig. 5 shows the comparison between the productions of 1-alcohols produced by oxidizing the n-alkanes with six to eight carbon atoms. For the bacterial suspensions Alk and H5, a decrease in the number of carbon atoms in the n-alkanes reduces the productions of the 1-alcohols. For the bacterial suspension H9, a decrease in the number of carbon atoms in the n-alkanes increases the productions of the 1-alcohols.

As described above, the productions of the oxidized compounds converted from the n-alkanes using the novel P450 monooxygenase are different from those converted from the n-alkanes using the P450 monooxygenases prepared using the known P450 gene CYP15313A for the arms of hybrid genes. That is, the novel P450 monooxygenase has specificity and specific activity to substrates different from those of other monooxygenases.

### [Example 15] Experiment of converting alkene by use of fused P450 monooxygenases

A bioconversion experiment was performed using the bacterial suspensions H3, H5, H7, H9, H11, and Alk prepared in Example 14 in such a manner that 1-octene, which is one of alkenes (unsaturated linear hydrocarbons), is used as a substrate. In particular, 0.25 ml of the substrate was added to 1 ml of each bacterial suspension and the mixture was subjected to a reaction at 20°C for 24 hours. Hydrochloric acid was added to the resulting mixture such that the mixture had a final hydrochloric acid concentration of 0.2 N, whereby the reaction was terminated. To the mixture, 0.25 ml of hexane was added. A hexane extract fraction obtained from this mixture was analyzed with a gas chromatography mass spectrometer, whereby 1,2-epoxyoctane produced by the oxidation of the substrate was identified and the amount thereof was determined. Table 4 shows the amounts (contents) of the oxidized compound produced by the oxidation using the bacterial suspensions.

**[Table 4]**

| (Substrate) Oxide | Bacterial Suspension Alk | Bacterial Suspension H3 | Bacterial Suspension H5 | Bacterial Suspension H7 | Bacterial Suspension H9 | Bacterial Suspension H11 |
|---|---|---|---|---|---|---|
| (1-Octene) 1,2-Epoxyoctane | 1122±129 ppm | 657t33 ppm | 1197±26 ppm | 115±14 ppm | 630t61 ppm | 2±1 ppm |

Table 4 shows that the hybrid P450 monooxygenases catalyze the epoxidation of 1-octene, which is one of unsaturated hydrocarbons.

### [Example 16] Experiment of converting cyclic hydrocarbon by use of fused P450 monooxygenases

A bioconversion experiment was performed using the bacterial suspensions H3, H5, H7, H9, H11, and Alk prepared in Example 14 in such a manner that cyclohexane, which is one of cyclic hydrocarbons (cycloalkanes), is used as a substrate. In particular, 0.25 ml of the substrate was added to 1 ml of each bacterial suspension and the mixture was subjected to a reaction at 20°C for 24 hours. Hydrochloric acid was added to the resulting mixture such that the mixture had a final hydrochloric acid concentration of 0.2 N, whereby the reaction was terminated. To the mixture, 0.25 ml of hexane was added. A hexane extract fraction obtained from this mixture was analyzed with a gas chromatography mass spectrometer, whereby cyclohexanol produced by the oxidation of the substrate was identified and the amount thereof was determined. Table 5 shows the amounts (contents) of the oxidized compound produced by the oxidation using the bacterial suspensions.

**[Table 5]**

| (Substrate) Oxide | Bacterial Suspension Alk | Bacterial Suspension H3 | Bacterial Suspension H5 | Bacterial Suspension H7 | Bacterial Suspension H9 | Bacterial Suspension H11 |
|---|---|---|---|---|---|---|
| (Cyclohexane) Cyclohexanol | 453±64 ppm | 117±7 ppm | 299±37 ppm | 7±2 ppm | 77±11 ppm | 290±1 ppm |

Table 5 shows that the hybrid P450 monooxygenases catalyze the alcoholization of cyclohexane, which is one of cyclic hydrocarbons.

### [Example 17] Experiments of converting aromatic hydrocarbons by use of fused P450 monooxygenases

The bioconversion of aromatic hydrocarbons (alkyl aromatics and alkenyl aromatics) were performed using the bacterial suspensions H5, H9, and Alk prepared in Example 14 and a bacterial suspension Non that was prepared in such a manner that plasmid vectors pAlkRED were transformed into strains of Escherichia coli BL21-AI and the strains were cultured without treatment for protein expression. The substrates were n-butylbenzene and 4-phenyl-1-butene. Each substrate was added to 1 ml of each bacterial suspension such that the mixture had a final substrate concentration of 1 mM. The mixture was subjected to a reaction at 20°C for eight hours. Hydrochloric acid was added to the resulting mixture such that the mixture had a final hydrochloric acid concentration of 0.2 N, whereby the reaction was terminated. To the mixture, 0.25 ml of ethyl acetate was added. An ethyl acetate extract fraction obtained from this mixture was analyzed for composition with a gas chromatography mass spectrometer. In the experiment using n-butylbenzene, the ethyl acetate extract fraction was treated with a sililating agent, TMSI-H (GL Science), such that a derivative was produced. The resulting fraction was analyzed for composition.

Fig. 6 shows the analysis of the fractions obtained by the reaction of n-butylbenzene. The fractions obtained from the bacterial suspensions H5, H9, and Alk contain 4-phenyl-1-butanol produced by the oxidation of n-butylbenzene. In contrast, the fraction obtained from the bacterial suspension Non containing no P450 monooxygenase contain no oxidized compound derived from n-butylbenzene. This experiment is the first to demonstrate the conversion of n-butylbenzene into 4-phenyl-1-butanol by the use of the P450 monooxygenases belonging to the CYP153 family.

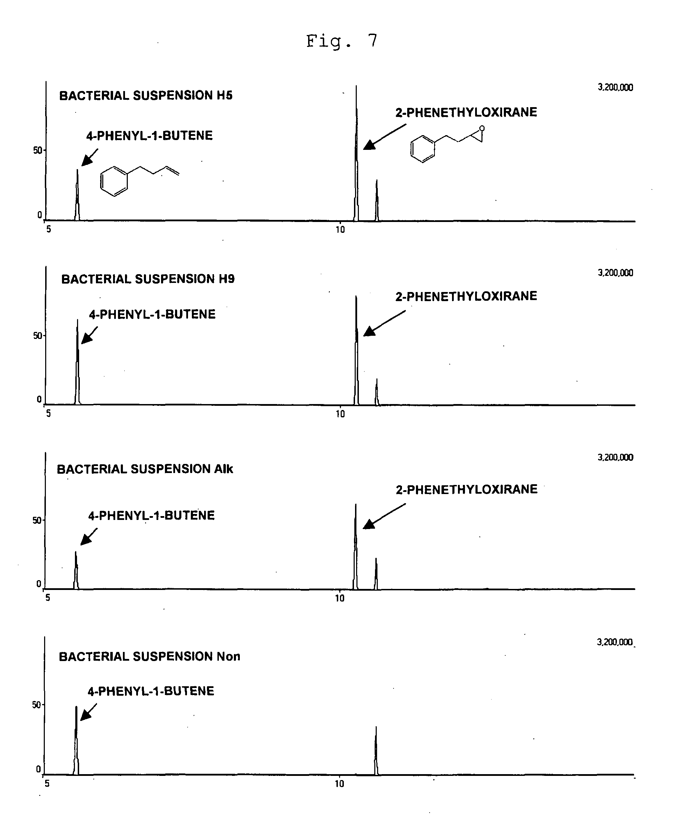

Fig. 7 shows the analysis of the fractions obtained by the reaction of 4-phenyl-1-butene. The fractions obtained from the bacterial suspensions H5, H9, and Alk contain 2-phenethyl-oxirane produced by the oxidation of 4-phenyl-1-butene. In contrast, the fraction obtained from the bacterial suspension Non containing no P450 monooxygenase contain no oxidized compound derived from 4-phenyl-1-butene. This experiment is the first to demonstrate the conversion of 4-phenyl-1-butene into 2-phenethyl-oxirane by the use of the P450 monooxygenases belonging to the CYP153 family.

### [Example 18] Preparation of artificial fusion enzyme containing P450 gene originating from Pseudomonas putida

The following fragment was PCR-amplified from a genomic DNA extracted from a strain of Pseudomonas putida ATCC 17453 using Primer 3 (5'-GACTAGTCATATGACGACTGAAACCATACA -3', SEQ ID NO: 75) and Primer 4 (5'-GGGAATTCTACCGCTTTGGTAGTCGCCGGA-3', SEQ ID NO: 76): a 1.3 kb DNA fragment containing a P450cam gene (Nucleotide sequence of the Pseudomonas putida cytochrome P450cam gene and its expression in Escherichia coli., J. Biol. Chem., 261: 1158-1163, 1986 and NCBI accession No. M12546) originating from Pseudomonas putida. The underlined sequences indicate an NdeI site (Primer 3) and an EcoRI site (Primer 4). A termination codon of the P450 gene was removed. The amplified 1.3 kb DNA fragments were cleaved at the NdeI and EcoRI sites and then subcloned into the NdeI-EcoRI sites of the plasmid pRED. The resulting plasmid is hereinafter referred to as pCAMRED. A plasmid pETP450cam for comparison was prepared by cloning the P450cam gene into the NdeI-EcoRI sites of a vector pET21a.

### [Example 19] Analysis of functions of fused P450 monooxygenase

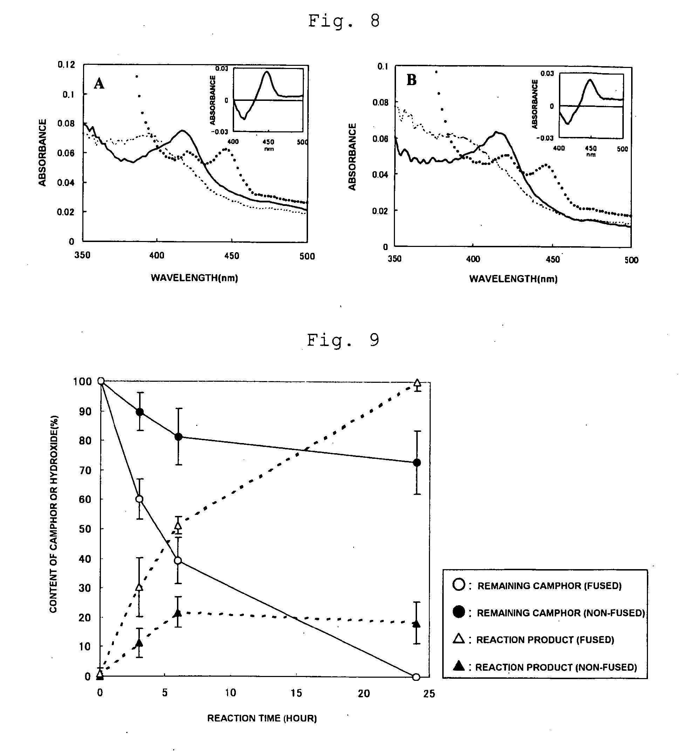

The plasmid pCAMRED prepared in Example 18 was transformed into a strain of Escherichia coli BL21(DE3) and the resulting strain was cultured in 3 mL of an LB liquid culture medium (containing 100 µg/ml of Ampicillin) at 30°C for 16 hours. Subsequently, 1 mL of a culture liquid obtained from the medium was added to 50 mL of an M9-glucose culture medium (containing 100 µg/ml of Ampicillin) and the culture was continued until the OD600 value reached about 0.8. IPTG serving as an inducer was added to the culture liquid such that the culture liquid had an IPTG concentration of 0.2 mM. The culture was further continued at 25°C for 16 hours. The cultured strains were collected and then suspended in 10 mL of a 10 mM phosphate buffer (pH 7.0). The suspension was subjected to ultrasonic disruption. The resulting suspension was subjected to centrifugal separation (10000xg for 20 minutes), whereby a cell-free extract was obtained from a supernatant portion of the suspension. Into two cuvettes, 0.8 ml of the cell-free extract was pipetted. After carbon monoxide was introduced into the sample-side cuvette, 5 to 10 mg of sodium thiosulfate was placed into both cuvettes. The cell-free extract in each cuvette was agitated and then subjected to spectrum analysis using light with a wavelength of 400 to 500 nm, whereby the cell-free extract was investigated if the cell-free extract contained an active P450 monooxygenase. Camphor serving as a substrate was added to 0.8 ml of the cell-free extract such that the mixture had a camphor concentration of 0.5 mM. The mixture was subjected to spectrum analysis. As shown in Fig. 8A, an artificial fused P450 monooxygenase has a spectrum pattern similar to that of a non-fused P450cam monooxygenase (Fig. 8B). That is, peaks are shifted from 420 nm to 450 nm because the P450 and P450cam monooxygenases in a reduced state are bound to carbon monoxide (dotted lines shown in Fig. 8). The CO difference spectra measured in the presence or absence of carbon monoxide have a maximum absorbance at 450 nm (inserted graphs in Fig. 8). In the spectrum of the mixture of the cell-free extract and the substrate, a maximum peak is shifted to 390 nm (a broken line in Fig. 8A). These show that the artificial fusion of the P450 monooxygenase and a reductase domain causes no adverse effects (the binding inhibition of the substrate due to steric hindrance or the like) and the fused P450cam monooxygenase has enzymatic activity.

### [Example 20] Conversion of camphor by use of Escherichia coli producing fused P450cam monooxygenase

A strain of Escherichia coli BL21 (DE3) containing the plasmid pCAMRED was cultured in the same manner as that described in Example 19. The cultured strains were subjected to gene expression. The resulting strains were collected and then suspended in 5 mL of a 10 mM phosphate buffer (pH 7.0). Camphor serving as a substrate for a P450cam monooxygenase was added to the suspension such that the mixture had a final camphor concentration of 0.5 mM. The strains were incubated at 25°C for 24 hours. After three, six, or 24 hours elapsed since the start of the reaction, each sample was obtained from the reaction solution. A liquid extract containing the substrate and a reaction product (5'-exo-hydroxide) was obtained from the reaction solution using ethyl acetate and then analyzed with a gas chromatography mass spectrometer (GC-MS, Shimadzu QP-5050), whereby identification was performed. As shown in Fig. 9, in the strains containing an artificial fusion enzyme gene, the sample obtained after 24 hours elapsed since the start of the reaction contains no camphor but camphor 5'-exo-hydroxide. In contrast, in strains of Escherichia coli BL21 (DE3) containing a non-fused plasmid pETP450cam (containing no RhF reductase domain) for comparison, about 20% of camphor is consumed during hydroxylation. The vertical axes of the graph in Fig. 9 show the consumption of the substrate or the accumulation of the reaction product. The content of the substrate at the start of the reaction is defined as 100% and the theoretical content of the reaction product at the complete consumption of the substrate is defined as 100%.

### [Example 21] Fusion of P450alk gene originating from Alcanivorax borkumensis

A 1.3 kb DNA fragment containing a P450alk (CYP153A13a) gene originating from Alcanivorax borkumensis was PCR-amplified from a genomic DNA extracted from a strain of Alcanivorax borkumensis SK2 (DSM11573) using Primer 5 (5'-CACTAGTCATATGTCAACGAGTTCAAGTACA-3', SEQ ID NO: 73) and Primer 6 (5'-CAGCACCAATTGTTTTTTAGCCGTCAACTT-3', SEQ ID NO: 74). The underlined sequences indicate an NdeI site (Primer 5) and an MfeI site (Primer 6). A termination codon of the P450 gene was removed. The amplified 1.3 kb DNA fragments were cleaved at the NdeI and MfeI sites and then subcloned into the NdeI-EcoRI sites of the plasmid pRED prepared in Example. The resulting plasmid is hereinafter referred to as pAlkRED. The nucleotide sequence of the P450alk (CYP153A13a) gene originating from Alcanivorax borkumensis SK2 (DSM11573) is set forth in SEQ ID NO: 58.

A plasmid pETP450alk for comparison was prepared by cloning only the P450alk gene into the NdeI-EcoRI sites of a vector pET21a.

### [Example 22] Conversion of alkane by use of Escherichia coli producing fused P450alk monooxygenase

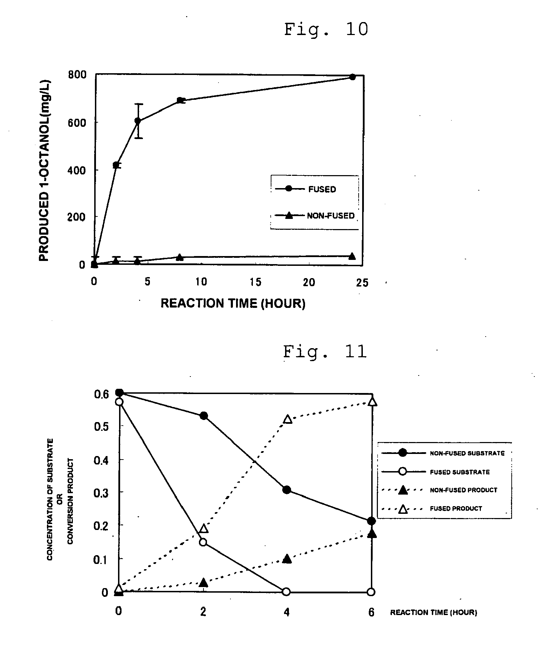

Strains of Escherichia coli BL21 (DE3) containing the plasmid pAlkRED or pETP450alk were cultured in the same manner as that described in Example 19. The cultured strains were subjected to gene expression. The resulting strains were collected and then suspended in 5 mL of 10 mM phosphate buffers (pH 7.0). To 1.5 mL of each suspension, 0.5 mL of octane serving as a substrate for a P450cam monooxygenase was added. The strains were incubated at 20°C for 24 hours. Samples were obtained from the reaction solution until 24 hours elapsed since the start of the reaction. A liquid extract containing a reaction product (1-octanol) was obtained from the reaction solution using octane and then analyzed with a gas chromatography mass spectrometer (GC-MS, Shimadzu QP-5050), whereby identification and determination were performed. Fig. 10 shows that the sample obtained from the reaction solution prepared using the strains containing the plasmid pAlkRED for expressing an artificial fusion enzyme gene contains 790.8 ppm of 1-octanol. In contrast, the sample obtained from the reaction solution prepared using the strains containing the non-fused plasmid pETP50cam (containing no RhF reductase domain) for comparison contains 40.3 ppm of 1-octanol. The content of 1-octanol in the sample obtained from the reaction solution prepared using these strains is about 5% of that in the sample obtained from the reaction solution prepared using those strains in which the artificial fusion enzyme gene has been expressed.

### [Example 23] Fusion of P450bzo gene obtained from environmental DNA sample

Uchiyama T, et al. have obtained a P450bzo gene from an environmental metagenome library by the SIGEX method (Uchiyama T, et al. Substrate-induced gene-expression screening of environmental metagenome libraries for isolation of catabolic genes, Nat. Biotechnol., 23: 88-93, 2005 and GenBank code: ABI86504). The P450bzo gene was subcloned. A 1.2 kb DNA fragment located close to the N-terminus of the P450,gene amplified using Primer 7 (5'-CGATATACATATGTTCAGTTTTGACCCCTAT-3', SEQ ID NO: 77) and Primer 8 (5'-GGATTGCGTTTGACGAATTTCACAAA-3', SEQ ID NO: 78). A 110 bb DNA fragment located close to the C-terminus of the P450 gene amplified using Primer 9 (5'-GGGCTCACTTTGTGAAATTCGTCAA3', SEQ ID NO: 79) and Primer 10 (5'-GGGAATTCCGAAACGGCCAATTCCAG-3', SEQ ID NO: 80). The 20th T in Primer 8 and the 15th A in Primer 9 are mutations introduced to remove an EcoRI site of the gene. There were no substitutes due to the mutations in amino acid sequences. The amplified 1.2 kb and 110 bp DNA fragments were mixed together. Full-length P450bzo gene fragments were prepared from the mixture by PCR using Primers 7 and 10. The underlined sequences in Primers 7 and 10 indicate an NdeI site and an EcoRI site. A termination codon of the P450 gene was removed. The amplified 1.3 kb fragments were cleaved at the NdeI and EcoRI sites and then subcloned into the NdeI-EcoRI sites of the plasmid pRED prepared in Example. The resulting plasmid is referred to as pBzoRED.

A plasmid pETP450bzo for comparison was prepared by cloning the P450bzo gene into the NdeI-EcoRI sites of an Escherichia coli vector pET21a.

### [Example 24] Conversion of 4-hydroxybenzoic acid by use of Escherichia coli producing fused P450bzo monooxygenase

Strains of Escherichia coli BL21 (DE3) containing the plasmid pBzoRED or pETP450bzo were cultured in the same manner as that described in Example 19. The cultured strains were subjected to gene expression. The resulting strains were collected and then suspended in 5 mL of 10 mM phosphate buffers (pH 7.0). To each suspension, 4-hydroxybenzoic acid serving as a substrate for a P450bzo monooxygenase was added. The strains were incubated at 20°C for six hours. After two, four, or six hours elapsed since the start of the reaction, each sample was obtained from the reaction solution. A liquid extract containing the substrate and a reaction product (3,4-dihydroxybenzoic acid) was obtained from the reaction solution using ethyl acetate and then analyzed with a gas chromatography mass spectrometer (GC-MS, Shimadzu QP-5050), whereby identification were performed. Fig. 11 shows that the sample obtained from the reaction solution prepared using the strains containing the plasmid pBzoRED for expressing an artificial fusion enzyme gene contains no substrate but about 0.6 mM of 3,4-dihydroxybenzoic acid, which is a product of hydroxylation, because of the rapid conversion of the substrate, the sample being obtained after four hours elapsed since the start of the reaction. In contrast, the sample obtained from the reaction solution prepared using the strains containing the non-fused plasmid pETP450bzo (containing no RhF reductase domain) for comparison has a substrate concentration (2.5 mM) that is half of that of that sample and also has a product concentration of about 0.1 mM, the sample being obtained after four hours elapsed since the start of the reaction.

### [Example 25] Fusion of P450SU-1 or P450SU-2 gene originating from Streptomyces griseolus

A 1.3 kb DNA fragment containing a P450SU-1 (CYP105A1) or P450SU-2 (CYP105B1) gene (Genes for two herbicide-inducible cytochromes P-450 from Streptomyces griseolus, J. Bacteriol., 172: 3335-45, 1990 and NCBI accession No. M32238 or M32239) originating from Streptomyces griseolus was PCR-amplified from a genomic DNA extracted from a strain of Streptomyces griseolus ATCC 11796 using a pair of Primer 11 (5'-GGACTCCATATGACCGATACCGCCACGACG-3', SEQ ID NO: 81) and Primer 12 (5'-CTGAATTCCCAGGTGACCGGGAGTTCGTTGAC-3', SEQ ID NO: 82) or a pair of Primer 13 (5'-GGACTCCATATGACGACCGCAGAACGCACC-3', SEQ ID NO: 83) and Primer 14 (5'-CTGAATTCCCAGGCGATCGGCAGCGAGTGGAC-3', SEQ ID NO: 84). The underlined sequences indicate NdeI sites (Primers 11 and 13) and EcoRI sites (Primers 12 and 14). A termination codon of the P450 gene was removed. The amplified 1.3 kb DNA fragments were cleaved at the NdeI and EcoRI sites and then inserted into the NdeI-EcoRI sites of the vector pRED, prepared in Example 12, for expressing function, whereby plasmids for expressing a fused P450SU-1 or P450SU-1 gene were prepared. The prepared plasmids are hereinafter referred to as pSU-1RED or pSU-2RED. A plasmid pETP450SU-1 and plasmid pETP450SU-2 for comparison were prepared by inserting only the P450SU-1 or P450SU-2 gene into the NdeI-EcoRI sites of a vector pET21a.

### [Example 26] Conversion of 7-ethoxycoumarin by use of Escherichia coli producing fused P450SU-1 or P450SU-2 monooxygenase

Strains of Escherichia coli BL21 (DE3) containing the plasmid pSU-1RED, pSU-2RED, pETP450SU-1, or pETP450SU-2 were cultured in the same manner as that described in Example 19. The cultured strains were subjected to gene expression. To culture media for culturing the strains, 7-ethoxycoumarin serving as a substrate for a P450SU-1 monooxygenase and a P450SU-2 monooxygenase was added simultaneously with the gene expression such that the culture media had a final 7-ethoxycoumarin concentration of 1 mM. The strains were incubated at 20°C for 74 hours. Each liquid extract containing the substrate and a reaction product (7-ethoxycoumarin) was obtained from a reaction solution, obtained from each culture medium, using ethyl acetate and then analyzed by thin-layer chromatography. The analysis showed that the extracts obtained from the reaction solutions prepared using the strains containing the plasmid pSU-1RED or pSU-2RED for expressing an artificial fusion enzyme gene contained 7-ethoxycoumarin, which was a reaction product. In contrast, the extract obtained from the reaction solution prepared using the strains containing the non-fused plasmid pETP450SU-1 contained no reaction product. Although the extract obtained from the reaction solution prepared using the strains containing the non-fused plasmid pETP450SU-2 contained a small amount of the reaction product, the content of the reaction product in this extract was less than that in the extract obtained from the reaction solution prepared using the strains containing the plasmid pSU-2RED.

This example shows that a P450 monooxygenase originating from actinomycete can be used for a function expression system according to the present invention.

### [Example 27] Isolation and fusion of P450HB153 gene

Nocardiaceae strain Hou_blue is a novel gram-positive bacterium and has been isolated from soil (oil-contaminated soil obtained in Niigata-ken) obtained from an oil field located in Nishiyama-cho, Niigata-ken on the basis of the ability to utilize an alkane, which is a petroleum hydrocarbon. The Hou_blue strain was deposited with Department of Biotechnology (2-5-8, Kazusa Kamatari, Kisarazu-shi, Chiba-ken), National Institute of Technology and Evaluation, on June 8, 2004 and the deposit number thereof was NITE P-3.

The following gene was isolated from the Nocardiaceae Hou_blue strain: a novel P450 gene (hereinafter referred to as a P450HB153 gene (the nucleotide sequence thereof is set forth in SEQ ID NO: 86 and the amino acid sequence of a P450 protein encoded by the gene is set forth in SEQ ID NO: 85)) belonging to the CYP153 family. A 1.3 kb DNA fragment containing the P450HB153 gene was PCR-amplified from a genomic DNA extracted from the strain using Primer Hou-1 (5'-GTAGGCCATATGAACGTAATCGGTGCAGGT-3', SEQ ID NO: 87) and Primer Hou-2 (5'-CATGAATTCGACCTGTTCCATCTCGGTCTC-3', SEQ ID NO: 88). The underlined sequences indicate an NdeI site and an EcoRI site. A termination codon of the P450HB153 gene was removed. The amplified 1.3 kb DNA fragments were cleaved at the NdeI and EcoRI sites and then inserted into the NdeI-EcoRI sites of the vector pRED prepared in Example 12, whereby a plasmid pHB153-Red was prepared.

### [Example 28] Conversion of 2-n-butylbenzofuran by use of Escherichia coli producing fused P450HB153 monooxygenase

An experiment of biochemically converting 2-n-butylbenzofuran was performed using a strain of Escherichia coli BL21 (DE3) containing a plasmid pHB153-Red. That is, the plasmid pHB153-Red prepared in Example 27 was transformed into a strain of Escherichia coli BL21(DE3) and the resulting strain was cultured in 3 mL of an LB liquid culture medium (containing 100 µg/ml of Ampicillin) at 30°C for 16 hours. Subsequently, 1 mL of a culture liquid obtained from the medium was added to 50 mL of an M9-glucose culture medium (containing 100 µg/ml of Ampicillin) and the culture was continued until the OD600 value reached about 0.8. IPTG serving as an inducer was added to the culture liquid such that the culture liquid had an IPTG concentration of 0.2 mM. The culture was further continued at 25°C for 16 hours. The culture liquid was subjected to centrifugal separation (8000rpm for 5 min.), whereby the cultured strains were precipitated. After a supernatant portion was removed from the culture liquid, 10 mL of a 50 mM phosphate buffer (pH 7:0) was added to the resulting culture liquid and the mixture was then vigorously stirred. The cultured strains were recovered from the mixture into 50 ml Falcon tubes. To each Falcon tube, 1.74 mg of the substrate dissolved in a small amount of DMDS was added (a final substrate concentration of 1 mM). The resulting strains were subjected to shaking culture at 20°C for 24 hours.

From 10 of the Falcon tubes, 100 ml (corresponding to 1 L of the original culture liquid) of a suspension containing the culture liquid used to convert 2-n-butylbenzofuran was recovered. The suspension was mixed with ethyl acetate and the mixture was partitioned. The ethyl acetate phase was recovered and then concentrated. The ethyl acetate extract (18.6 mg) was subjected to TLC analysis (a developer containing hexane and ethyl acetate (5 : 1). The analysis showed the presence of a product (hereinafter referred to as Compound 28-1) with an Rf value of 0.21. The ethyl acetate extract was purified by silica gel chromatography (1 cm diameter, 20 cm length, a developer containing hexane and ethyl acetate (5 : 1), whereby pure Compound 28-1 (0.4 mg) was obtained. The analysis of Compound 28-1 by EI-MS showed a molecular ion peak Mϕ at m/z 190 (substrate + one oxygen atom). The analysis of Compound 28-1 by ¹H NMR spectrometry showed that a signal corresponding to an aromatic ring portion thereof was identical to that of the substrate. The analysis also showed that there was no terminal methyl group of the substrate and Compound 28-1 had three methylene groups and an oxymethylene group with a triplet at δ 3.70. The analysis of the DQF-COSY spectrum thereof showed that these four methylene groups were linked to one another. From the analysis of the DQF-COSY spectrum and the analysis by EI-MS, it was confirmed that this compound had a hydroxyl group inserted into a terminal methyl group. That is, Compound 28-1 was identified to be 4-benzofran-2-yl-butan-1-ol.

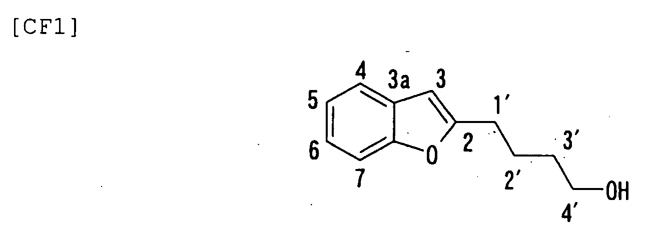

This compound is specified in CAS and is the first produced by microbial conversion.

**[Table 6]**

| 400-MHz ¹H NMR Data of Compound 28-1 (4-benzofuran-2-yl-butan-1-ol) (in CDCl₃) | | |
|---|---|---|
| Position | δ_{H} | |
| 3 | 6.40 (s) | |
| 4 | 7.47 (d7.0) | |
| 5 | 7.21 | |
| 6 | 7.21 | |
| 7 | 7.40 (d 7.1) | |
| 1' | 2.82 (t) | |
| 2' | 1.85 (m) | |
| 3' | 1.70 (m) | |
| 4' | 3.70 (t 6.3) | |

### Industrial Applicability

The present invention provides a method for efficiently isolating a novel P450 gene from a sample, such as an environmental sample, containing various microbial nucleic acids. P450 produced from the P450 gene obtained by the method has specificity and activity to various substrates and can be used for the oxidation of various low-molecular-weight organic compounds such as alkanes, alkenes, cyclic hydrocarbons (cycloalkanes), and aromatics (alkyl or alkenyl aromatics).

This specification includes the matters specified in the specifications and/or drawings of Japanese patent applications (No. 2004-326139, No. 2005-83019, and No. 2005-83122) to which this application claims priority. The contents of the publications, patents, and patent applications cited in this specification are incorporated herein by reference.

## Claims

1. An oligonucleotide, having substantially a primer or probe function, for isolating a P450 gene fragment, containing a nucleotide sequence encoding part or all of the amino acid sequence set forth in SEQ ID NO: 51 or 52 or a nucleotide sequence complementary thereto.

2. An oligonucleotide, having substantially a primer or probe function, for isolating a P450 gene fragment, containing the nucleotide sequence set forth in any one of SEQ ID NOS: 53 to 56 or part or all of a nucleotide sequence complementary thereto.

3. A method for isolating a P450 gene fragment, comprising a step of using the oligonucleotide according to Claim 1 or 2 as at least one primer.

4. A method for isolating a P450 gene fragment, comprising a step of extracting a nucleic acid from a sample and a step of performing nucleic acid amplification using the nucleic acid and the oligonucleotide according to Claim 1 or 2 as a template and a primer, respectively.

5. A method for preparing a hybrid P450 gene, comprising a step of isolating a P450 gene fragment using the oligonucleotide according to Claim 1 or 2 as at least one primer and a step of adding a 5'-terminal region and 3'-terminal region of a known P450 gene to the 5'-terminus and 3'-terminus, respectively, of the isolated P450 gene.

6. The method according to Claim 5, wherein the known P450 gene encodes the amino acid sequence set forth in SEQ ID NO: 57.

7. A kit for isolating a P450 gene, comprising:
(a) the oligonucleotide according to Claim 1 or 2;
(b) a DNA fragment containing a 5'-terminal region of a known P450 gene; and
(c) a DNA fragment containing a 3'-terminal region of a known P450 gene.

8. A gene encoding the protein specified in any one of Items (a) and (b) below:
(a) a protein containing the amino acid sequence set forth in any one of SEQ ID NOS: 1 to 25; and
(b) a protein, serving as P450, containing an amino acid sequence prepared by removing one or more amino acid residues from the amino acid sequence set forth in any one of SEQ ID NOS: 1 to 25, replacing one or more amino acid residues of this amino acid sequence with other residues, or adding one or more amino acid residues to this amino acid sequence.

9. A gene encoding a protein, serving as P450, containing the nucleotide sequence set forth in any one of SEQ ID NOS: 26 to 50.

10. An expression vector containing the gene according to Claim 8 or 9.

11. A protein as specified in any one of Items (a) and (b) below:
(a) a protein containing the amino acid sequence set forth in any one of SEQ ID NOS: 1 to 25; and
(b) a protein, serving as P450, containing an amino acid sequence prepared by removing one or more amino acid residues from the amino acid sequence set forth in any one of SEQ ID NOS: 1 to 25, replacing one or more amino acid residues of this amino acid sequence with other residues, or adding one or more amino acid residues to this amino acid sequence.

12. A method for producing a protein serving as P450, comprising a step of transforming the expression vector according to Claim 10 into a host cell and then culturing the host cell and a step of recovering the protein according to Claim 11 from the cultured cells.

13. A method for producing an oxidized compound, comprising a step of subjecting a substrate to a reaction in the presence of the protein according to Claim 11 to produce an oxidized compound.

14. A fused cytochrome P450 monooxygenase containing a peptide which is linked to the C-terminus of a P450 protein with a linker portion disposed therebetween and which has the same function as that of a reductase domain contained in a cytochrome P450 monooxygenase originating from Rhodococcus sp. strain NCIMB 9784.

15. The fused cytochrome P450 monooxygenase according to Claim 14, wherein the peptide is (a) one containing the amino acid sequence set forth in SEQ ID NO: 70; (b) one containing an amino acid sequence prepared by adding one or more amino acid residues to the amino acid sequence set forth in SEQ ID NO: 70; removing one or more amino acid residues from this amino acid sequence, or replacing one or more amino acid residues of this amino acid sequence with other residues; or (c) one encoded by a DNA hybridized with a DNA containing the nucleotide sequence set forth in SEQ ID NO: 69 or a DNA complementary to this DNA under stringent conditions.

16. The fused cytochrome P450 monooxygenase according to Claim 14 or 15, wherein the linker portion is (d) a peptide containing the amino acid sequence set forth in SEQ ID NO: 68; (e) a peptide containing an amino acid sequence prepared by adding one or more amino acid residues to the amino acid sequence set forth in SEQ ID NO: 68; removing one or more amino acid residues from this amino acid sequence, or replacing one or more amino acid residues of this amino acid sequence with other residues; or (f) a peptide encoded by a DNA hybridized with a DNA containing the nucleotide sequence set forth in SEQ ID NO: 67 or a DNA complementary to this DNA under stringent conditions.

17. The fused cytochrome P450 monooxygenase according to any one of Claims 14 to 16, wherein the P450 protein originates from a bacterium.

18. The fused cytochrome P450 monooxygenase according to any one of Claims 14 to 16, wherein the P450 protein is identical to the protein according to Claim 11 or a protein containing the amino acid sequence set forth in SEQ ID NO: 85.

19. A DNA encoding the fused cytochrome P450 monooxygenase according to any one of Claims 14 to 18.

20. A microorganism containing the DNA according to Claim 19.

21. A method for producing a fused cytochrome P450 monooxygenase, comprising a step of culturing the microorganism according to Claim 20 and a step of recovering a fused cytochrome P450 monooxygenase from the cultured microorganisms or cells thereof.

22. A method for producing an oxidized compound, comprising a step of subjecting a substrate to a reaction in the presence of the fused cytochrome P450 monooxygenase according to any one of Claims 14 to 18 to produce an oxidized compound.

23. The method according to Claim 22, wherein the substrate is n-hexane, n-heptane, n-octane, n-decane, 1-octene, cyclohexane, n-butylbenzene, 4-phenyl-1-butene, or 2-n-butylbenzofran and the oxidized compound is 1-hexanol, 1-heptanol, 1-octanol, 1-decanol, 1,2-epoxyoctane, cyclohexanol, 4-phenyl-1-butanol, 2-phenethyloxirane, or 4-benzofran-2-yl-butane-1-ol.

24. A gene encoding the protein specified in any one of Items (a) and (b) below:
(a) a protein containing the amino acid sequence set forth in SEQ ID NO: 85; and
(b) a protein, serving as P450, containing an amino acid sequence prepared by removing one or more amino acid residues from the amino acid sequence set forth in SEQ ID NO:85, replacing one or more amino acid residues of this amino acid sequence with other residues, or adding one or more amino acid residues to this amino acid sequence.

25. A gene encoding a protein, serving as P450, containing the nucleotide sequence set forth in SEQ ID NO: 86.
